(19) Europäisches Patentamt
European Patent Office
Office européen des brevets

(11) EP 4 745 155 A1

(12) **EUROPEAN PATENT APPLICATION**
published in accordance with Art. 153(4) EPC

(43) Date of publication:
**20.05.2026 Bulletin 2026/21**

(21) Application number: **24839709.3**

(22) Date of filing: **08.07.2024**

(51) International Patent Classification (IPC):
***C07K 14/78*** (2006.01) ***A61K 9/14*** (2006.01)
***A61K 33/08*** (2006.01) ***A61K 47/02*** (2006.01)
***A61K 47/42*** (2017.01) ***A61L 15/08*** (2006.01)
***A61L 15/12*** (2006.01) ***A61L 15/18*** (2006.01)
***A61L 15/32*** (2006.01) ***A61L 27/02*** (2006.01)
***A61L 27/12*** (2006.01) ***A61L 27/22*** (2006.01)
***A61P 35/00*** (2006.01)

(52) Cooperative Patent Classification (CPC):
**A61K 9/14; A61K 33/08; A61K 47/02; A61K 47/42; A61L 15/08; A61L 15/12; A61L 15/18; A61L 15/32; A61L 27/02; A61L 27/12; A61L 27/22; A61P 35/00; C07K 14/78**

(86) International application number:
**PCT/JP2024/024585**

(87) International publication number:
**WO 2025/013818 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.07.2023 JP 2023112745**

(71) Applicant: **National Institute for Materials Science**
**Tsukuba-shi, Ibaraki 305-0047 (JP)**

(72) Inventor: **TAGUCHI Tetsushi**
**Tsukuba-shi, Ibaraki 305-0047 (JP)**

(74) Representative: **J A Kemp LLP**
**80 Turnmill Street**
**London EC1M 5QU (GB)**

# (54) POWDER CONTAINING PARTICLES OF GELATIN DERIVATIVE AND APPLICATION OF SAME IN MEDICAL TREATMENT

(57) The present invention provides a powder which is capable of forming a tissue covering material that is capable of exhibiting a further function while having high adhesive strength to living tissues. The powder includes first particles that each contain a crosslinked gelatin derivative, and second particles that are different from the first particles and can provide a further function. The gelatin derivative has a structure represented by formula (1). In formula (1), Gltn represents a residue of gelatin, L represents a single bond or a divalent linking group, $R^1$ represents a hydrocarbon group having 1 to 20 carbon atoms, and $R^2$ represents a hydrogen atom or a hydrocarbon group having 1 to 20 carbon atoms.

[Chemical Formula 1] $GltnNH\text{-}L\text{-}CHR^1R^2$ • • • (1)

EP 4 745 155 A1

**Description**

TECHNICAL FIELD

The present invention relates to a powder containing particles of a gelatin derivative, and medical applications of the same.

BACKGROUND ART

Gelatin is excellent in biocompatibility and biodegradability, and is therefore used for various medical applications. The inventor of the present application has reported, in PATENT LITERATURE 1, particles of a crosslinked gelatin derivative suitable for a wound dressing, an anti-adhesion material, a hemostatic material, and the like. The particles can be applied to, for example, biological tissues by spraying, and are gelled and adhered to the tissues to function as a tissue covering material.

CITATION LIST

PATENT LITERATURE

PATENT LITERATURE 1: WO2020/137903 A

SUMMARY OF INVENTION

TECHNICAL PROBLEM

The crosslinked gelatin derivative particles of PATENT LITERATURE 1 exhibit excellent adhesion to biological tissues as a tissue covering material. If a further function can be incorporated into the tissue covering material while maintaining this adhesion property, broader medical applications can be anticipated. Furthermore, it would be desirable to further increase the adhesion strength to biological tissues.

The present invention solves the above problem. That is, the present invention provides, in one embodiment, a powder capable of forming a tissue covering material that is capable of exhibiting a further function while exhibiting high adhesion strength to biological tissues. The present invention also provides, in another embodiment, a tissue covering material with a higher adhesion strength to biological tissues.

SOLUTION TO PROBLEM

The present inventors, as a result of diligently investigated to achieve the above objectives, provides the following powder and medical applications thereof.

[1] A powder comprising first particles containing a crosslinked gelatin derivative; and

second particles which are different from the first particles and can provide a further function,
wherein the gelatin derivative has a structure represented by the formula (1) described below.

[2] The powder according to [1], wherein the second particles contain a particulate pharmaceutical agent.
[3] The powder according to [2], wherein the pharmaceutical agent is hydrophobic or positively or negatively charged.
[4] The powder according to any one of [1] to [3], wherein a ratio of a mass of the second particles (W2) to a mass of the first particles (W1) (W2/W1) is 0.1/50 to 60/50.
[5] The powder according to [4], wherein the ratio of a mass of the second particles (W2) to a mass of the first particles (W1) (W2/W1) is 0.1/50 to 40/50.
[6] The powder according to [4], wherein the ratio of a mass of the second particles (W2) to a mass of the first particles (W1) (W2/W1) is 1/50 to 60/50.
[7] The powder according to any one of [1] to [6], wherein an average particle diameter of the first particles is 0.1 μm to 100 μm.
[8] The powder according to any one of [1] to [7], wherein an average particle diameter of the second particles is 5 nm to 100 μm.
[9] The powder according to [8], wherein the average particle diameter of the second particles is 10 nm to 100 nm.
[10] The powder according to any one of [1] to [9], wherein a ratio of the average particle diameter of the second

particles to the average particle diameter of the first particles (D2/D1) is 0.001 to 0.1.

[11] The powder according to any one of [1] to [10], wherein the powder has a water contact angle of 50 degrees or more as the water contact angle was measured one second after water is added dropwise.

[12] The powder according to any one of [2] to [11], wherein the pharmaceutical agent is at least one selected from the group consisting of an anticancer agent, an immunosuppressant, a growth factor, a cell differentiation inducer, an antimetabolite, a growth factor inhibitor, an immune checkpoint inhibitor, an antibiotic, an anti-inflammatory agent, and a protein preparation.

[13] The powder according to [12], wherein the pharmaceutical agent contains at least one selected from the group consisting of a hydrophobic anticancer agent, a hydrophobic immunosuppressant, and a hydrophobic cell differentiation inducer.

[14] The powder according to [13], wherein the crystallinity of the hydrophobic anticancer agent is 30 to 50% as determined by XRD crystallographic analysis.

[15] The powder according to [13] or [14], wherein the hydrophobic anticancer agent is selected from the group consisting of cabazitaxel, paclitaxel, docetaxel, cisplatin, carboplatin, nedaplatin, oxaliplatin, tamoxifen, anastrozole, glivec, 5-fluorouracil (5-FU), floxuridine, leuprolide, flutamide, zoledronate, doxorubicin, vincristine, gemcitabine, streptozocin, vinorelbine, retinoic acid series, adriamycin, mitomycin, daunomycin, prednisone, testosterone, mitoxantrone, aspirin, salicylic acid, ibuprofen, naproxen, fenoprofen, indomethacin, phenyltazone, cyclophosphamide, celecoxib, valdecoxib, and nimesulide.

[16] The powder according to [14], wherein the hydrophobic immunosuppressant is selected from the group consisting of steroids (for example, hydrocortisone, cortisone acetate, prednisone, prednisolone, methylprednisolone, triamcinolone, dexamethasone, betamethasone), cyclosporine, mizoribine (bredinin), cyclophosphamide (endoxan), azathioprine (azanin), tacrolimus (FK506), sirolimus (rapamycin), deoxyspergualin (gusperimus hydrochloride), everolimus, ABT-578 (zotarolimus), basiliximab (an anti-IL-2 receptor monoclonal antibody), muromonab CD3 (an anti-CD3 monoclonal antibody), and arbulin (an anti-lymphocyte globulin).

[17] The powder according to [14], wherein the hydrophobic cell differentiation inducer is tamibarotene.

[18] The powder according to [12], wherein the antimetabolite is pemetrexed.

[19] The powder according to [12], wherein the growth factor inhibitor is bevacizumab.

[20] The powder according to [12], wherein the immune checkpoint inhibitor is nivolumab.

[21] The powder according to any one of [2] to [12], wherein the pharmaceutical agent contains a growth factor, and the powder further contains a cationic polymer.

[22] The powder according to [21], wherein the growth factor is selected from the group consisting of vascular endothelial growth factor (VEGF), basic fibroblast growth factor (bFGF), platelet-derived growth factor (PDGF), hepatocyte growth factor (HGF), transforming growth factor-$\beta$ (TGF-$\beta$), and Wnt7b.

[23] The powder according to any one of [1] to [22], wherein the second particles contain inorganic fine particles.

[24] The powder according to [23], wherein the second particles contain magnetic particles and an anticancer agent, preferably a hydrophobic anticancer agent.

[25] The powder according to [23], wherein the second particles contain calcium phosphate and a growth factor.

[26] A tissue covering material comprising the powder according to any one of [1] to [25].

[27] A cancer killing material comprising the powder according to [24].

[28] The cancer killing material according to [27], wherein the magnetic particles are iron oxide.

[29] A bone filling material comprising the powder according to [25].

[30] The powder according to [1], wherein the second particles are inorganic fine particles.

[31] The powder according to [30], wherein the second particles contain a metal.

[32] The powder according to [30] or [31], wherein a ratio of a mass of the second particles (W2) to a mass of the first particles (W1) (W2/W1) is 20/50 to 60/50.

[33] The powder according to [32], wherein the ratio of a mass of the second particles (W2) to a mass of the first particles (W1) (W2/W1) is 20/50 to 40/50.

[34] The powder according to [32], wherein the ratio of a mass of the second particles (W2) to a mass of the first particles (W1) (W2/W1) is 40/50 to 60/50.

[35] The powder according to any one of [30] to [34], wherein an average particle diameter of the first particles is 0.1 $\mu$m to 100 $\mu$m.

[36] The powder according to any one of [30] to [35], wherein an average particle diameter of the second particles is 5 nm to 100 $\mu$m.

[37] The powder according to [36], wherein the average particle diameter of the second particles is 10 nm to 100 nm.

[38] The powder according to any one of [30] to [37], wherein a ratio of the average particle diameter of the second particles to the average particle diameter of the first particles (D2/D1) is 0.001 to 0.1.

[39] The powder according to any one of [30] to [38], wherein the second particles are magnetic particles or calcium phosphate.

[40] The powder according to any one of [30] to [39], wherein the powder has a water contact angle of 50 degrees or more as the water contact angle was measured one second after water is added dropwise.

[41] A tissue covering material comprising the powder according to any one of [30] to [40].

[42] A cancer-killing material comprising the powder according to [39] or [40], wherein the second particles are magnetic particles.

[43] The cancer-killing material according to [42], wherein the magnetic particles are iron oxide.

[44] A bone filling material comprising the powder according to [39] or [40], wherein the second particles are calcium phosphate.

[45] A method for covering or filling a tissue, comprising topically administering an effective amount of the powder according to any one of [1] to [25] and [30] to [40] to a predetermined site of the tissue to form a gel.

[46] A method for covering or filling an excised site of a tissue, comprising the step of topically administering an effective amount of the powder according to any one of [1] to [25] and [30] to [40] to an excised site of a patient after surgery with tissue excision or its periphery to form a gel.

[47] The method according to [45] or [46], wherein the second particles contain an anticancer agent, and the tissue is tumor tissue.

[48] A method for treating cancer in a patient with a tumor, comprising the step of topically administering a therapeutically effective amount of the powder according to any one of [12] to [15], [23], and [24] to a tumor tissue or its periphery in the patient to form a gel, wherein the second particles contain an anticancer agent.

[49] The method according to [48], wherein the second particles further contain magnetic particles.

[51] A method for treating or alleviating inflammation in a patient suffering from ulcerative colitis, comprising the step of topically administering a therapeutically effective amount of the powder according to any one of [12], [13], and [16] to an inflamed site or its periphery in the patient to form a gel, wherein the second particles contain an immunosuppressant.

[52] A method for treating polycystic kidney disease in a patient with autosomal dominant polycystic kidney disease, comprising the step of topically administering a therapeutically effective amount of the powder according to any one of [12], [13], and [17] to a renal tissue or its periphery in the patient to form a gel, wherein the second particles contain a cell differentiation inducer.

[53] A method for promoting tissue regeneration in a patient after tissue removal, comprising the step of topically administering a therapeutically effective amount of the powder according to any one of [12], [13], and [18] to a tissue removal site or its periphery in the patient to form a gel, wherein the second particles contain a growth factor.

[54] A method for killing residual malignant pleural mesothelioma in a patient after removal of malignant pleural mesothelioma, comprising topically administering a therapeutically effective amount of the powder according to [12] to a cancer removal site or its periphery in the patient to form a gel, wherein the second particles contain an antimetabolite.

[55] A method for treating malignant pleural mesothelioma in a patient with malignant pleural mesothelioma, comprising the step of topically administering a therapeutically effective amount of the powder according to [12] to a cancer tissue or a cancer removal site tissue or its periphery in the patient to form a gel, wherein the second particles contain a growth factor inhibitor.

[56] A method for treating kidney cancer, non-small cell lung cancer, head and neck cancer, melanoma, Hodgkin's disease, or liver cancer in a patient with kidney cancer, non-small cell lung cancer, head and neck cancer, melanoma, Hodgkin's disease, or liver cancer, comprising the step of topically administering a therapeutically effective amount of the powder according to [12] to a cancer tissue or a cancer tissue removal site or its periphery in the patient to form a gel, wherein the second particles contain an immune checkpoint inhibitor.

[57] The method according to [45] to [56], wherein the gel has a water content of 50 to 70%.

[58] The method according to [45] to [57], wherein the gel has a pressure resistance of 70 mmHg or more.

## ADVANTAGEOUS EFFECTS OF INVENTION

The present invention provides a powder suitable for forming a tissue covering material. A tissue covering material formed by a powder according to one embodiment has high adhesion strength to biological tissues and can exhibit a further function. In addition, a tissue covering material formed by a powder according to another embodiment can exhibit higher adhesion strength to biological tissues.

## BRIEF DESCRIPTION OF DRAWINGS

FIG. 1 is a schematic diagram illustrating the configuration of a powder of one embodiment, which contains gelatin

derivative particles (C10MPs) and inorganic particles (SPIONs).

FIG. 2 is a schematic diagram illustrating an example of using the gel formed by hydration of a powder according to one embodiment as a tissue covering material (cancer killing material).

FIG. 3 shows enlarged diagrams (conceptual diagrams) of the region 2B shown in FIG. 2.

FIG. 4 shows enlarged diagrams (conceptual diagrams) of the region 2C shown in FIG. 2.

FIG. 5 is a schematic diagram illustrating a method for producing a powder containing gelatin derivative particles (C10MPs) and inorganic particles (SPIOSs) in Examples.

FIG. 6 shows SEM photographs of first particles (C10MPs), second particles (SPIONs), and raw material gelatin particles (OrgMPs) prepared in Examples.

FIG. 7 is a graph showing particle size distributions of first particles (C10MPs) and raw material gelatin particles (OrgMPs) prepared in Examples.

FIG. 8 is a graph showing a particle size distribution of second particles (SPIONs) prepared in Examples.

FIG. 9 shows microscopic photographs observing the hydration of a powder (SPION/C10MP) and a powder (SPION/OrgMP).

FIG. 10 shows the IR spectra of the gel (SPION/C10MP) and gel (SPION/OrgMP) formed after powder hydration.

FIG. 11 is a graph showing the storage moduli of the gel (OrgMP), gel (SPION/C10MP), and gel (SPION/OrgMP) formed after powder hydration.

FIG. 12 is a schematic diagram illustrating the interaction between components in the gel (SPION/C10MP) formed after powder hydration.

FIG. 13 is a schematic diagram illustrating an adhesion strength test for the gel formed after powder hydration in Examples.

FIG. 14 is a graph showing the results of the adhesion strength tests for the gel (OrgMP), gel (SPION/OrgMP), gel (C10MP), and gel (SPION/C10MP) formed after powder hydration.

FIG. 15 is a schematic diagram illustrating a pressure resistance test of the gel formed after powder hydration in Examples.

FIG. 16 is a graph showing the results of the pressure resistance tests for the gel (OrgMP), gel (SPION/OrgMP), gel (C10MP), and gel (SPION/C10MP) formed after powder hydration.

FIG. 17 is a graph showing the results of the heat generation tests of the gel (OrgMP), gel (SPION/OrgMP), gel (C10MP), and gel (SPION/C10MP) formed after powder hydration.

FIG. 18 is a graph showing the survival rates of cancer cells in an in vitro test using a gel formed by hydration of a powder (SPION/C10MP) as a cancer-killing material.

FIG. 19 is a graph showing the survival rate of normal cells in an in vitro test using a gel formed by hydration of a powder (SPION/C10MP) as a cancer killing material.

FIG. 20 is a graph showing therapeutic effects (change in cancer size) in an in vivo test using a gel formed by hydration of a powder (SPION/C10MP) as a cancer-killing material.

FIG. 21 is a graph showing therapeutic effects (mouse survival rate) in an in vivo test using a gel formed by hydration of a powder as a cancer-killing material.

FIG. 22 is a schematic diagram illustrating a preparation method, a configuration, and medical applications of a powder according to another embodiment, wherein the second particles contain a pharmaceutical agent.

FIG. 23 shows X-ray diffraction patterns of commercial PTX and reprecipitated PTX.

FIG. 24(a) are photographs of the water droplet shapes one minute after dropping water droplets onto a powder obtained by mixing PTX particles and C10MP particles (PTX/C10MP), OrgMP, C10MP, and a powder obtained by mixing PTX particles and OrgMP particles (PTX/OrgMP), and FIG. 24(b) shows the water contact angle of each particles or powder.

FIG. 25 shows the results of measuring the water content of colloidal gels formed with OrgMP, PTX/OrgMP, C10MP, and PTX/C10MP.

FIG. 26 is a schematic diagram illustrating a pressure resistance test of a gel formed after powder hydration.

FIG. 27(a) shows the results of measuring the pressure resistance of colloidal gels formed with OrgMPs, PTX/OrgMPs, C10MPs, and PTX/C10MPs. FIG. 27(b) schematically shows the internal structure of the formed colloidal gel.

FIG. 28 is a graph showing the proportions of PTX released from each of the colloidal gels formed with PTX/OrgMP and PTX/C10MP when the colloidal gels were immersed in PBS.

FIG. 29 is a schematic diagram showing the protocol of a test in which PTX/C10MP was injected into the peritoneum of a tumor-bearing mouse model.

FIG. 30 is a graph showing changes over time in tumor volume of each group in which PTX, OrgMP, C10MP, PTX/OrgMP, or PTX/C10MP was applied to the peritoneum of a tumor-bearing mouse model.

DESCRIPTION OF EMBODIMENTS

The description of the components described below may be made based on representative embodiments of the present invention, but the present invention is not limited to such embodiments.

As used herein, a numerical range expressed using "to" denotes a range including numerical values described before and after "to" as a lower limit value and an upper limit value, respectively.

In the notation of groups (atomic groups) in this specification, a notation that does not indicate substitution or non-substitution encompasses both those having substituents and those having no substituents, to the extent that the effect of the present invention is not impaired. For example, "alkyl group" encompasses not only alkyl groups without substituents (unsubstituted alkyl groups) but also alkyl groups with substituents (substituted alkyl groups). This applies equally to each compound.

The present invention relates to a powder comprising first particles containing a crosslinked gelatin derivative and second particles which are different from the first particles and can provide a further function, and therapeutic applications using the powder.

The powder can be produced by a very simple method in which the first particles and the second particles are separately prepared and mixed.

Hereinafter, powders according to various embodiments of the present invention will be described in detail.

<First particles>

Examples of the first particles include a powder disclosed in PATENT LITERATURE 1 (WO2020/137903 A), the contents of which are incorporated herein by reference. Specifically, the first particles contain a crosslinked gelatin derivative (hydrophobized gelatin), and the gelatin derivative has a structure represented by the following formula (1).

[Chemical Formula 1] GtlnNH-L-CHR$^1$R$^2$ • • • (1)

In the formula (1), Gltn represents a residue of gelatin, L represents a single bond or a divalent linking group, R$^1$ represents a hydrocarbon group having 1 to 20 carbon atoms, and R$^2$ represents a hydrogen atom or a hydrocarbon group having 1 to 20 carbon atoms.

The divalent linking group of L is not particularly limited, and examples thereof include -C(O)-, -C(O)O-, -OC(O)-, -O-, -S-, -N(R)- (R represents a hydrogen atom or a monovalent organic group (preferably a hydrocarbon group having 1 to 20 carbon atoms)), an alkylene group (preferably an alkylene group having 2 to 10 carbon atoms), an alkenylene group (preferably an alkenylene group having 2 to 10 carbon atoms), combinations thereof, and the like; and among them, -C(O)- is preferred. Therefore, L is preferably a single bond or -C(O)-.

*-CHR$^1$R$^2$ (* denotes a bonding position) is preferably bound to an ε-amino group of the gelatin of a raw material (raw material gelatin), and more preferably bound to an ε-amino group of lysine (Lys) in the gelatin. Methods of bonding *-CHR$^1$R$^2$ to an amino group, preferably the amino group of lysine via a linking group or without a linking group (directly in other words) include, for example, methods of utilizing a so-called reductive amination reaction (a method using aldehyde or ketone), the Schotten-Baumann reaction (a method using acid chloride), and the like.

The -NH- structure in the formula (1) can be detected, for example, by the presence of a band near 3300 cm$^{-1}$ in an FT-IR (Fourier transform infrared absorption) spectrum.

The hydrocarbon group having 1 to 20 carbon atoms is not particularly limited, and examples thereof include a chain hydrocarbon group having 1 to 20 carbon atoms, an alicyclic hydrocarbon group having 3 to 20 carbon atoms, an aromatic hydrocarbon group having 6 to 14 carbon atoms, and groups obtained by combining these groups.

When R$^2$ is a hydrocarbon group having 1 to 20 carbon atoms, R$^2$ may be the same as or different from R$^1$. Also, the alkyl groups of R$^1$ and R$^2$ may be linear or branched.

The chain hydrocarbon group having 1 to 20 carbon atoms is not particularly limited, and examples thereof include a methyl group, an ethyl group, a propyl group, a butyl group, a hexyl group, an octyl group (or a capryl group), a nonyl group (or a pelargonyl group), a decyl group, a dodecyl group (or a lauryl group), a tetradecyl group (or a myristyl group), and the like. Among them, R$^1$ is preferably an alkyl group having 1 to 13 carbon atoms, more preferably an alkyl group having 7 to 12 carbon atoms, further preferably an alkyl group having 8 to 11 carbon atoms, and particularly preferably an alkyl group having 9 to 11 carbon atoms because a powder having superior adhesion can easily be obtained. R$^2$ is not particularly limited, but is preferably a hydrogen atom.

Examples of the alicyclic hydrocarbon group having 3 to 20 carbon atoms include a cyclopropyl group, a cyclopentyl group, a cyclohexyl group, an adamantyl group, a norbornyl group, and the like.

The aromatic hydrocarbon group having 6 to 14 carbon atoms is not particularly limited, and examples thereof include a phenyl group, a tolyl group, a naphthyl group, and the like.

The group obtained by combining the above groups is not particularly limited, and examples thereof include

aralkyl groups having 6 to 12 carbon atoms such as a benzyl group, a phenethyl group, a naphthylmethyl group, a naphthylethyl group, and the like.

In addition, when the crosslinked gelatin derivative having the structure represented by the formula (1) is injected in vivo, as reported by the inventor of the present application in JP-B-7132465, the crosslinked gelatin derivative exhibits an angiogenesis promoting action on the cells around the injection site. The contents described in JP-B-7132465 are incorporated herein by reference. From the viewpoint of promoting angiogenesis and promoting adhesion to a tissue, the total carbon number of $-CHR^1R^2$ in the formula (1) is preferably 9 to 20, and more preferably 12 to 14. When the total number of carbon atoms is in this range, a gel formed by applying the powder in vivo exhibits excellent angiogenesis ability, and when the gel is used as a bone filling material that enables blood vessel induction, bone regeneration can be promoted.

As the gelatin derivative represented by the formula (1), at least one gelatin derivative selected from the group consisting of the following formula (2) and formula (3) is preferred, and the gelatin derivative represented by the formula (2) is more preferred.

[Chemical Formula 2] **$GltnNH-CHR^1R^2$** • • • (2)

[Chemical Formula 3]

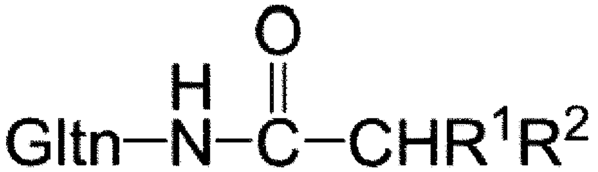 (3)

In the formula (2) and formula (3), the meaning of each symbol is the same as that already described in the formula (1), and preferred options are also the same as those already described in the formula (1).

As used herein, the "derivatization rate" is defined as a molar ratio of the content of imino groups to which a hydrophobic group (hydrocarbon group) is bonded in the gelatin derivative in relative to a content of amino groups in the raw material gelatin.

The derivatization rate of the gelatin derivative is not particularly limited, but is generally preferably 20 to 80 mol%, and more preferably 30 to 70 mol%. In other words, the imino group/amino group (molar ratio) in the obtained gelatin derivative is preferably 20/80 to 80/20, and more preferably 30/70 to 70/30.

In the present specification, the number of amino groups in the raw material gelatin and the number of amino groups in the gelatin derivative are quantified by a 2,4,6-trinitrobenzenesulfonic acid method (TNBS method), and the derivatization rate is calculated from the obtained values by the following formula.

Derivatization rate (mol%) = [Number of amino groups in raw material gelatin - Number of amino groups in gelatin derivative]/[Number of amino groups of raw material gelatin] $\times$ 100

Gelatins used as a raw material for the gelatin derivative (hereinafter, also referred to as "the raw material gelatin".) may be naturally-occurring ones or synthesized ones (including fermented gelatins, genetically modified gelatins, and the like), or may be gelatins obtained by subjecting naturally-occurring or synthesized gelatins to any treatments.

More specific examples thereof include naturally-occurring gelatins obtained from skin, bone, tendon, and the like of mammal, bird, fish, and the like, treated gelatins prepared by treating naturally-occurring gelatins with an acid or an alkali (heat-extracted as necessary), and the like.

Among them, alkali-treated gelatins are preferred because a powder having a superior effect of the present invention can be obtained.

In addition, when the powder is used for in vivo administration, for example, as a wound dressing or the like, it is preferable to use low-endotoxinized gelatin with reduced content of endotoxin. Such low-endotoxinized gelatin is not particularly limited, and any known low-endotoxinized gelatin can be used, and examples thereof include those described in JP-A-2007-231225, the contents of which are incorporated herein by reference.

Gelatins derived from mammal include gelatins derived from porcine and bovine. Gelatins derived from fish are not particularly limited, but among them, gelatins derived from cold-water fishes such as salmon, trout, cod, Alaska pollock, sea bream, tilapia, and tuna (hereinafter, also referred to as "cold-water fish-derived gelatins".) is preferred.

The cold-water fish-derived gelatins are linear polymers of two or more amino acids which have 190 or less imino acids, more specifically 80 or less hydroxyprolines and 110 or less prolines, per 1000 constituent amino acids. The cold-water fish gelatins are fluid at ordinary temperature, which is believed to be due to the number of hydroxyprolines being 80 or less or the number of prolines being 110 or less. Either of the conditions is deemed to allow a denaturation temperature to be approximately room temperature or lower, which leads to fluidity at ordinary temperature.

Sea bream gelatin has 73 hydroxyprolines and 108 prolines, and has a denaturation temperature of 302.5 K. Tilapia gelatin contains 82 hydroxyprolines and 110 prolines, and has a denaturation temperature of 309 K. On the other hand, porcine gelatin contains 95 hydroxyprolines and 121 prolines, and has a denaturation temperature of 316 K.

The cold-water fish-derived gelatins have amino acid sequences similar to those of gelatins derived from animals, and are readily decomposed by enzymes, and has high biocompatibility.

The molecular weight of the raw material gelatin is not particularly limited, but the weight-average molecular weight (Mw) thereof is preferably 5,000 to 100,000, more preferably 10,000 to 50,000, and further preferably 20,000 to 40,000. As used herein, the weight-average molecular weight means a weight-average molecular weight determined by gel permeation chromatography (GPC).

The first particles contain the crosslinked gelatin derivative described above. As used herein, the "crosslinked" means a crosslinked structure obtained by an irreversible crosslinking reaction, excluding reversible physical crosslinking structures. Therefore, the "crosslinked gelatin derivative" is a gelatin derivative having an irreversible crosslinked structure obtained through a crosslinking reaction induced by applying energy to the gelatin derivative via heat, light, energy rays, or the like, and/or by using a crosslinking agent. Typically, they occur through reactions between functional groups ($-NH_2$, -OH, -SH, -COOH, and the like) on the side chains of gelatin.

The first particles are only required to contain a crosslinked gelatin derivative, and may contain other components as long as the effect of the present invention is exhibited. The content of a crosslinked gelatin derivative in the first particles 10 is not particularly limited, but to readily obtain a powder exhibiting superior effects of the present invention, the crosslinked gelatin derivative is contained in preferably an amount of 90 mass% or more and more preferably an amount of 99 mass% or more with respect to the total mass of the first particles 10. Examples of the other components include a non-crosslinked gelatin derivative, a solvent, a buffering agent, a colorant, a preservative, an excipient, a pharmaceutical agent (such as an antithrombotic agent, an antibacterial agent, an anticancer agent, an immunosuppressant, or a growth factor), and the like. However, since the particles are typically produced by thermally crosslinking a gelatin derivative, other components contained inside the particles risk being aggregated, deformed, altered, or denatured during thermal crosslinking. For example, if inorganic fine particles or a pharmaceutical agent (second particles) are incorporated into the first particles, the first particles may be aggregated during thermal crosslinking to increase the particle diameter, leading to deterioration of the adhesion of a gel to tissues. In addition, when the inorganic fine particles and the pharmaceutical agent (second particles) are aggregated, deformed, altered, or denatured during thermal crosslinking, they may fail to fully perform their intended functions. For instance, as described later, using magnetic particles as the second particles enables heating of gel 40 by an external magnetic field, and this function can be applied to achieve localized death of cancer cells (see FIG. 4). However, when a magnetic powder in the gel 40 is aggregated, heating via the external magnetic field becomes difficult, so that sufficient cancer cell killing effects may not be achieved.

The average particle size of the first particles 10 may be, for example, 0.1 $\mu$m to 100 $\mu$m, 0.5 $\mu$m to 50 $\mu$m, 1 $\mu$m to 30 $\mu$m, or 1 $\mu$m to 10 $\mu$m. As used herein, the "average particle diameter" can be measured by a method described in Examples described later.

The method for producing the first particles is not particularly limited, and for example, the first particles can be produced by the method described in PATENT LITERATURE 1 (WO2020/137903 A).

<Second particles>

As shown in FIG. 1, a powder 30 of one embodiment contains inorganic fine particles (the second particles) 20 together with particles (the first particles) 10 containing a crosslinked gelatin derivative. The first particles 10 and the second particles 20 are separate individuals, and one is not contained in the other. The powder 30 of the present embodiment includes a mixture of two types of particles (the first particles 10 and the second particles 20).

The second particles 20 constituting the powder 30 are not particularly limited as long as they are inorganic fine particles, and inorganic fine particles having various functions can be used, and various applications according to the functions, particularly medical applications, can be anticipated.

For example, magnetic particles may be used as the second particles 20. In case a powder 30 containing the first particles 10 and the magnetic particles (the second particles) 20 is applied to biological tissues, a gel 40 containing the magnetic particles (the second particles) 20 can be formed, and the gel 40 can be heated by applying an external magnetic field. It is known that cancer cells are vulnerable to heat compared to normal cells. The powder 30 can be applied as a cancer-killing material capable of selectively destroying cancer cells while minimizing damage to normal cells (see FIG. 4).

The magnetic particles are not particularly limited, but those having low biological toxicity are preferred.

Examples thereof include iron oxide (for example, $Fe_3O_4$, $Fe_2O_3$), ferrite, chromium oxide, gold, cobalt, and the like, and among them, iron oxide is preferred in that it is composed of a metal abundant in the body when ionized.

For example, calcium phosphate may be used as the second particles 20. Calcium phosphate such as hydroxyapatite is used as a main component of an artificial bone because it has osteoconductive property (ability to promote bone ingrowth around tit). Therefore, the powder 30 containing the first particles 10 and the calcium phosphate (the second particles) 20 can be selectively applied to the affected area (fracture site or the like) by spraying or the like and also used as a bone filling material.

Examples of the other inorganic fine particles include β-tricalcium phosphate (β-TCP), α-tricalcium phosphate (α-TCP), and octacalcium phosphate (OCP) as calcium phosphate having bone inducibility.

From the viewpoint of further improving the adhesive strength of a gel 40 to tissues, the second particles 20 preferably contain a metal. The strength of the gel 40 is improved by the interaction between the metal-containing fine particles and functional groups of the gelatin derivative (for example, a carboxy group), so that the adhesive strength of the gel 40 can be further enhanced. The metal contained in the second particles 20 may be determined based on an intended function of the second particles 20, but is preferably a metal having low biological toxicity. Examples of the metal include sodium (Na), potassium (K), magnesium (Mg), calcium (Ca), iron (Fe), aluminum (Al), vanadium (V), chromium (Cr), cobalt (Co), nickel (Ni), copper (Cu), zinc (Zn), and the like.

Examples of the other inorganic particles include titanium oxide and cerium oxide.

As illustrated in FIG. 22, a powder 60 of another embodiment includes first particles 10 containing a crosslinked gelatin derivative and second particles 50 containing or consisting of a particulate pharmaceutical agent. The second particles 50 are individuals independent from the first particles 10, and one is not contained in the other. Therefore, the powder 60 of this embodiment is also a mixture containing the first particles 10 and the second particles 50. The first particles 10 of this embodiment are similar to the first particles of the aforementioned embodiment.

The gelatin derivative constituting the first particles 10 is hydrophobic and has functional groups such as a carboxyl group. Therefore, in the present embodiment, when the first particles are hydrated to form a colloidal gel, the pharmaceutical agent interacts with the gelatin derivative through hydrophobic interactions or electrostatic interactions to be incorporated into the gel, and is locally sustained-released from the gel adhered to the tissue.

In this embodiment, the pharmaceutical agent is not particularly limited, and the pharmaceutical agent may be a low molecular weight compound or a high molecular weight bioactive substance. In a preferred embodiment, the pharmaceutical agent is selected from hydrophobic, or negatively or positively charged pharmaceutical agents. The hydrophobic pharmaceutical agents interact hydrophobically with the first particles 10, which is hydrophobic, thereby improving the tissue adhesion and pressure resistance of a gel formed from the powder 60. Since the colloidal gel formed by gelatin is negatively charged, the positively charged pharmaceutical agents can electrostatically bind to the colloidal gel. The negatively charged pharmaceutical agents can electrostatically bind to the colloidal gel via cationic additives when used together with them.

Examples of the pharmaceutical agent include an anticancer agent, an immunosuppressant, a growth factor, a cell differentiation inducer, an antimetabolite, a growth factor inhibitor, an immune checkpoint inhibitor, an antibiotic, an anti-inflammatory agent, and a protein preparation, and these pharmaceutical agents may be used alone or in combination of two or more thereof.

The anticancer agent is not particularly limited, but a hydrophobic anticancer agent is preferred, and examples thereof include cabazitaxel, paclitaxel, docetaxel, cisplatin, carboplatin, nedaplatin, oxaliplatin, tamoxifen, anastrozole, glivec, 5-fluorouracil (5-FU), floxuridine, leuprolide, flutamide, zoledronate, doxorubicin, vincristine, gemcitabine, streptozocin, vinorelbine, retinoic acid series, adriamycin, mitomycin, daunomycin, prednisone, testosterone, mitoxantrone, aspirin, salicylic acid, ibuprofen, naproxen, fenoprofen, indomethacin, phenyltazone, cyclophosphamide, celecoxib, valdecoxib, and nimesulide.

The immunosuppressant is not particularly limited, but a hydrophobic immunosuppressant is preferred, and examples thereof include low molecular weight compounds such as steroids (for example, hydrocortisone, cortisone acetate, prednisone, prednisolone, methylprednisolone, triamcinolone, dexamethasone, betamethasone), cyclosporine, mizoribine (bredinin), cyclophosphamide (endoxan), azathioprine (azanin), tacrolimus (FK506), sirolimus (rapamycin), deoxyspergualin (gusperimus hydrochloride), everolimus, and ABT-578 (zotarolimus), and polymeric active substances such as basiliximab (an anti-IL-2 receptor monoclonal antibody), muromonab CD3 (an anti-CD3 monoclonal antibody), and arbulin (an anti-lymphocyte globulin).

The cell differentiation inducer is not particularly limited, but a hydrophobic cell differentiation inducer is preferred, and examples thereof include tamibarotene.

The antimetabolite is not particularly limited, and examples thereof include pemetrexed. The growth factor inhibitor is not particularly limited, and examples thereof include bevacizumab.

The immune checkpoint inhibitor is not particularly limited, and examples thereof include nivolumab.

Examples of the growth factor include vascular endothelial growth factor (VEGF), basic fibroblast growth factor (bFGF), platelet-derived growth factor (PDGF), hepatocyte growth factor (HGF), transforming growth factor-β (TGF-β),

Wnt7b, and the like.

Biologically active macromolecules such as growth factors carry either positive or negative charges. Since the colloidal gel formed by the gelatin constituting the first particle carries a negative charge, positively charged active molecules can electrostatically bind to the colloidal gel. Furthermore, negatively charged, biologically active macromolecules such as growth factors can electrostatically bind to the colloidal gel via cationic additives when used together with them. Examples of cationic additives include cationic polymers such as polylysine, chitosan, and polyallylamine.

For pharmaceutical agents that are low molecular weight compounds, the release rate can be adjusted by the degree of crystallinity. Specifically, the higher the crystallinity, the slower the release rate, while the lower the crystallinity, the faster the release rate. Therefore, it is possible to set an appropriate release rate in combination with the hydrophobicity of pharmaceutical agents. For example, when the pharmaceutical agent is a hydrophobic anticancer agent, immunosuppressant, or cell differentiation inducer, setting the crystallinity to 30-50% allows for sustained release over a period of several months to half a year. The degree of crystallinity can be adjusted by changing the temperature and the concentration of the pharmaceutical agent when the pharmaceutical agent is reprecipitated. Specifically, a solution of an pharmaceutical agent is prepared and a poor solvent is added dropwise thereto to reprecipitate the pharmaceutical agent by setting the solution temperature to a range of 10 to 40°C, and the concentration of the pharmaceutical agent to 0.1 to 10 w/v%, and slowly adding the poor solvent dropwise, so that the crystallinity can be set to 30 to 50%. When the crystallinity is reduced, precipitation is performed at a lower temperature for a shorter time. When the crystallinity is increased, precipitation may be performed at a higher temperature for a longer time.

The average particle diameter of the second particles 20 and 50 may be appropriately adjusted according to the type (function) thereof, and is preferably, for example, 5 nm to 100 μm or 10 nm to 10 μm. When the average particle diameter of the second particles 20 and 50 falls within the above range, it is possible to impart further functions to a gel 40 while maintaining excellent adhesion properties without significantly affecting the interaction between the hydrophobized gelatins. In addition, when the second particles are magnetic particles, the average particle diameter is more preferably 5 nm to 100 nm, 10 nm to 100 nm, or 10 nm to 20 nm from the viewpoint of enhancing the heating efficiency. Furthermore, in case the second particles are a particulate pharmaceutical agent, the average particle diameter is more preferably 10 nm to 100 nm, 20 nm to 100 nm, or 50 nm to 100 nm from the viewpoint of inclusion in a gel. As used herein, the "average particle diameter" can be measured by a method described in Examples described later.

The second particles 20 contained in the powder 30 may be used alone as a single type, or may be used as a mixture of two or more types as long as the effect of the present invention is exhibited. Also, the second particles 20 may be produced by a known method, or a commercially available product may be used.

The second particles may contain, for example, both a pharmaceutical agent and inorganic fine particles. For instance, the second particles contain an anticancer agent as an pharmaceutical agent, and may further contain magnetic particles. In this case, the anticancer agent and the magnetic particles may be integrated to constitute a particle, or may be individual particles. The latter is preferred in this embodiment.

Alternatively, the second particles may contain a growth factor such as VEGF or Wnt7b as a pharmaceutical agent and further contain calcium phosphate. Even in this case, the growth factor and calcium phosphate may be integrated to constitute a particle, or may be individual particles. Also in this embodiment, the latter is preferred.

<Powder>

Powders 30 and 60 contain a mixture of particles (the first particles) 10 containing the aforementioned crosslinked gelatin derivative and functional particles (the second particles) 20 and 50. The ratio of the mass of the second particles 20 and 50 (W2) to the mass of the first particles 10 (W1) (W2/W1) is not particularly limited, but it is preferable to select an appropriate ratio according to the type of functional particles (the second particles). For instance, in case the second particles are or contain functional inorganic particles 20, the ratio (W2/W1) is preferably 20/50 to 60/50, 20/50 to 50/50, or 20/50 to 40/50 from the viewpoint of increasing the adhesive strength of the gel 40 formed from the powder 30. In case the second particles are or contain magnetic particles 20, the ratio (W2/W1) is preferably 40/50 to 60/50 from the viewpoint of increasing heat generation efficiency. In case the second particles are particulate pharmaceutical agent 50 or contain the pharmaceutical agent, the ratio (W2/W1) is preferably 0.1/50 to 60/50, 0.1/50 to 50/50, or 0.1/50 to 40/50 from the viewpoint of increasing the adhesive strength of the gel 70 formed from the powder 60. In particular, when the second particles 50 are or contain a growth factor, a physiologically active protein, or an antibody, the ratio (W2/W1) is preferably 0.1/50 to 60/50.

The powders 30 and 60 may consist of the first particles 10 and the second particles 20 and 50, and may contain other components different from the first particles 10 and the second particles 20 and 50 as long as the effect of the present invention is exhibited. The ratio of the total mass of the first particles 10 and the second particles 20 and 50 in the powders 30 and 60 may be, for example, 90 mass% or more, 99 mass% or more, or 100 mass%. The other components that may be contained in the powder 30 are not particularly limited, and examples thereof include a non-crosslinked gelatin derivative, a solvent, a buffering agent, a colorant, a preservative, an excipient, a pharmaceutical agent (such as an antithrombotic

agent, an antibacterial agent, an anticancer agent, an immunosuppressant, or a growth factor), and the like.

The ratio of an average particle diameter of the second particles 20 and 50 (D2) to an average particle diameter of the first particles 10 (D1) (D2)/(D1) is not particularly limited, and may be, for example, 0.001 to 100, 0.001 to 1, or 0.001 to 0.1. In addition, from the viewpoint of increasing the adhesive strength of gels 40 and 70 to tissues, the particle diameter of the second particles 20 and 50 is preferably smaller than the particle diameter of the first particles (that is, the ratio (D2)/(D1) is less than 1, and may be, for example, 0.001 to 0.1). In case the second particles 20 are magnetic particles, the ratio (D2)/(D1) is preferably 0.001 to 0.1 or 0.001 to 0.01 from the viewpoint of increasing heat generation efficiency. In case the second particles are a particulate pharmaceutical agent, the ratio (D2)/(D1) is preferably 0.001 to 0.1 or 0.01 to 0.1 from the viewpoint of adhesion and delivery property.

Methods for producing the powders 30 and 60 according to the present embodiment are not particularly limited. For example, the first particles and the second particles may be prepared and mixed at a desired ratio by known methods to produce the powders.

The resulting powders 30, 60 contain, for example, the first particles and the second particles in the ratio (W2/W1) and the ratio (D2)/(D1) described above, whereby the gels formed are hydrophobic as a whole. Therefore, the powders 30 and 60 may have a water contact angle of 50 degrees or more, preferably a water contact angle of 60 degrees or more, and more preferably a water contact angle of 70 degrees or more, depending on the structures of the gelatin derivatives and the numerical values of the ratio (W2/W1) and the ratio (D2)/(D1). In addition, the powders 30 and 60 usually have a water contact angle of 90 degrees or less, and often have a water contact angle of 80 degrees or less. As used herein, the "water contact angle" is measured by a method described in Examples described later.

The water contact angle is an index indicating the hydrophobicity of the gelatin derivatives and thus the gels formed thereby, and in the range of the water contact angle described above, the adhesion strength to tissues and the pressure resistance are increased, and in case the first particles contain a hydrophobic compound as a pharmaceutical agent, these strengths are further improved.

Furthermore, the colloidal gels formed from powders 30 and 60, due to its hydrophobicity, have lower water contents than those of the colloidal gels formed from powders comprising the first particle composed of non-hydrophobized gelatin, and for example, the colloidal gels have a water content of 50 to 70%, preferably a water content of 55 to 65%. As used herein, the "water content" is determined by a method described in Examples described later.

Such a decrease in water content means that the inside of the gels is dense due to hydrophobic interaction, and it is understood that this improves the pressure resistance of the gels.

In fact, the colloidal gels 40 and 70 formed from the powders 30 and 60 have a pressure resistance of 70 mmHg or more, preferably 80 mmHg or more in a pressure resistance test. Also, in the colloidal gel 70 formed from a powder 60 in which the second particles contain a hydrophobic pharmaceutical agent, the pressure resistance is further improved by hydrophobic interaction with the hydrophobic pharmaceutical agent. Specifically, the colloidal gel 70 has a pressure resistance of 90 mmHg or more, preferably 100 mmHg or more. Further, a powder 60 in which the second particles do not contain a hydrophobic pharmaceutical agent usually has a pressure resistance of 100 mmHg or less, and the powder 60 in which the second particles contain a hydrophobic pharmaceutical agent usually has a pressure resistance of 120 mmHg or less. As used herein, the "pressure resistance" is determined by a method described in Examples described later.

<Application of powder>

When the powders 30 and 60 according to the present embodiment are applied (for example, sprayed) onto a biological tissues in a dry state, the first particles 10 absorb blood and moisture to gelate, and exhibit excellent adhesion to the biological tissues while retaining the functional second particles 20, 50. Applying these properties , the powders 30 and 60 of the present embodiment can be used as a tissue covering material or tissue filling material having various functions. For example, effective amounts of the powders 30, 60 can be topically administered to an excised site of a patient after surgery with tissue excision or its periphery to form a gel, thereby covering or filling the excised site of the tissue. Furthermore, while the powders can certainly be used as a tissue covering material, powders of some embodiments can be used as a cancer killing material or a bone filling material.

Further, similarly to the powder disclosed in PATENT LITERATURE 1 (WO2020/137903 A), it can also be used as a wound dressing, an anti-adhesion material, a hemostatic material, and the like. The application amount can be appropriately adjusted according to the intended use and the application site.

In case magnetic particles are used as the second particles 20, the gel formed from the powder 30 can be used not only as a wound dressing material but also as a cancer-killing material. In addition, after the gel adheres to the tissue, the hydrophobic groups that were exposed on the surface of the first particles are submerge into the gel in aqueous environments, causing the exposed surface to gradually become hydrophilic, which reduces the tendency for adhesion to other tissues and allows the gel to function as an anti-adhesion material. Further, since spray application is possible, the gel can be used for wound area not only in laparotomy but also in endoscopic surgery. For example, when the powder 30 of this embodiment is applied (sprayed) to an affected area of a living tissue (for example, a damaged area after cancer tumor

resection by an endoscope) (FIG. 2(a)), the powder 30 applied on the tissue is hydrated to form a gel 40 (FIG. 2(b)). At this time, it is inferred that a hydrophobic interaction between the first particles 10 (FIG. 3(a)), a hydrophobic interaction between the first particles 10 and the tissue (cell membrane, cell matrix) (FIG. 3(b)), and an interaction between the second particles and the first particles (for example, a coordinate bond between metal-containing fine particles and carboxy groups) (FIG. 3(c)) occur, and the gel 40 of the present embodiment has improved bulk strength and adhesion (adhesive force) to the tissue, and further has enhanced stability in water (FIG. 3(d)).

In this manner, by applying the powder 30 to an affected area (wound area) in endoscopic cancer tumor resection surgery, it is possible to cover the wound and to suppress postoperative adhesion, and further, by applying an external magnetic field, it is also possible to kill cancer cells that may remain (FIG. 4).

Also, as described above, in case calcium phosphate is used as the second particles, the powder can be selectively applied to an affected area (fracture site or the like) by spraying, syringe, or the like, and used as a bone filling material. In addition, it can be used as a bone filling material for a jawbone defect area at the time of oral surgery or in the implant application.

As shown in FIG. 22, in case the second particles 50 contain an effective amount of an pharmaceutical agent, the second particles 50 can be used not only as a wound dressing material but also for treatment of various diseases depending on the type of pharmaceutical agent, and can further function as an anti-adhesion material. For example, in case the second particles 50 contain an effective amount of an anticancer agent, a therapeutically effective amount of the powder 60 can be topically administered to a tumor tissue or its periphery in a patient with a tumor to form a gel, thereby treating cancer in the patient. Furthermore, by locally administering powder 60 to the affected area (wound site) during endoscopic cancer tumor resection surgery, the wound can be covered, postoperative adhesions can be suppressed, and, moreover, the sustained release of anticancer agents can eliminate any potentially remaining cancer cells. In these embodiments, the anticancer agent is preferably a hydrophobic anticancer agent. In addition, the second particles may contain magnetic particles together with an anticancer agent, and in this case, an additive or synergistic effect with the anticancer agent can be expected for eliminating cancer cells by applying an external magnetic field to heat the tumor tissue.

In case the second particles contain an effective amount of an immunosuppressant, a therapeutically effective amount of the powder is topically administered to an inflamed site or its periphery in a patient suffering from ulcerative colitis to form a gel, whereby inflammation in the patient can be treated or alleviated. In this embodiment, the immunosuppressant is preferably a hydrophobic immunosuppressant.

In case the second particles contain a cell differentiation inducer, a therapeutically effective amount of the powder is topically administered to a renal tissue or its periphery in a patient with autosomal dominant polycystic kidney disease to form a gel, whereby the polycystic kidney disease of the patient can be treated. In this embodiment, the cell differentiation inducer is preferably a hydrophobic cell differentiation inducer.

In case the second particles contain a growth factor, a therapeutically effective amount of the powder is topically administered to a removal site or its periphery in a patient after tissue excision to form a gel, whereby tissue regeneration of the patient can be promoted. As described above, a growth factor may be positively or negatively charged and in case of containing a growth factor negatively charged, the second particles preferably contain a cationic polymer as well. The tissue to be excised may contain tumor tissue. In addition, in case the second particles contain a growth factor such as VEGF or Wnt7b as a pharmaceutical agent and a gel formed from the powder is used as a bone filling material, calcium phosphate may be further contained. In this embodiment, bone formation is expected to be further promoted.

In case the second particles contain an antimetabolite, a therapeutically effective amount of the powder is topically administered to a cancer removal site or its periphery in a patient after removal of malignant pleural mesothelioma to form a gel, whereby residual malignant pleural mesothelioma in the patient can be treated. In this embodiment, the antimetabolite is preferably a hydrophobic antimetabolite.

In case the second particles contain a growth factor inhibitor, a therapeutically effective amount of the powder is topically administered to a cancer removal site or its periphery in a patient after removal of malignant pleural mesothelioma to form a gel, whereby residual malignant pleural mesothelioma in the patient can be treated. In this embodiment, the growth factor inhibitor is preferably a hydrophobic growth factor inhibitor.

In case the second particles contain an immune checkpoint inhibitor, a therapeutically effective amount of the powder is topically administered to a cancer removal site or its periphery in a patient with kidney cancer, non-small cell lung cancer, head and neck cancer, melanoma, Hodgkin’s disease, or liver cancer to form a gel, whereby the kidney cancer, non-small cell lung cancer, head and neck cancer, melanoma, Hodgkin’s disease, or liver cancer of the patient can be treated. In this embodiment, the immune checkpoint inhibitor is preferably a hydrophobic immune checkpoint inhibitor.

## EXAMPLES

Hereinafter, the present invention is described in further detail based on Examples. The materials, amounts, percentages, conditions of process, process procedures and the like shown in the following Examples can be changed as

appropriate, unless departing from the spirit of the present invention. Accordingly, the present invention is not to be construed as limited by the following Examples.

1. First particles

<Synthesis of gelatin derivative (hydrophobized gelatin)>

An amino group of raw material gelatin (hereinafter, referred to as "Org-ApGltn" as appropriate) was reacted with decanal to form a Schiff base, and then the obtained Schiff base was reduced to a stable secondary amine with a reducing agent to synthesize a hydrophobized gelatin (hereinafter, referred to as "C10-ApGltn" as appropriate) (see FIG. 5). The details are described below.

100 g of Alaska pollock-derived gelatin (Org-ApGltn) (weight-average molecular weight (Mw): 38,552 Da, amino group content: 339 μmol/g, manufactured by Nitta Gelatin Inc.) was dissolved in 105 mL of ultrapure water, and two equivalent amount of decanal (67.8 mmol, manufactured by Tokyo Chemical Industry Co., Ltd.) with respect to the amino groups of Org-ApGltn (339 μmol/g) were added to the solution along with ethanol (manufactured by JUNSEI CHEMICAL CO., LTD.) while stirring at 50°C to form an imine bond between the decanal and the amino groups of Org-ApGltn. After stirring the mixed liquid at the same temperature for 1 hour, 2-picoline borane (50.85 mmol, manufactured by JUNSEI CHEMICAL CO., LTD.) was added to the mixed liquid along with ethanol to reduce the imine. The resulting mixed solution (Org-ApGltn concentration: 20 mass/volume%, water: ethanol = 105: 45 mL) was stirred at 50°C for 17 hours to proceed the reaction. Thereafter, the reaction solution (150 mL) was added dropwise to 1,500 mL of cold ethanol (-7 to 4°C) to purify C10-ApGltn. The resulting reprecipitated material was washed with 1,500 mL of ethanol (3 times for 1 hour) to remove unreacted decanal and 2-picoline borane. Thereafter, the resulting material was dried in a vacuum for 3 days to obtain C10-ApGltn at a yield of 92.9 mass%.

The introduction of decyl groups into the obtained C10-ApGltn could be confirmed by Fourier transform infrared spectroscopy and $^1$H-NMR. In addition, the number of amino groups in the raw material gelatin and the number of amino groups in the hydrophobized gelatin were quantified by a 2,4,6-trinitrobenzenesulfonic acid method (TNBS method), and the derivatization rate (hereinafter, referred to as "DS" as appropriate) of C10-ApGltn was calculated from the obtained values. The DS was 47 mol%.

<Production of first particles>

0.5 g of the gelatin derivative obtained by the above method was weighed into a vial with scale (50 mL), and 7.5 mL of MilliQ (registered trademark) water was added thereto. Next, after dissolving the gelatin derivative with a water bath at 50°C, the solution was diluted up to 10 mL with MilliQ water. At this time, the content (final concentration) of the gelatin derivative in the gelatin solution was adjusted to 5 mass/volume%.

Next, ethanol (EtOH) was added dropwise with stirring by a stirrer bar at room temperature until the gelatin solution became cloudy to obtain a gelatin solution containing intermediate particles. Next, the gelatin solution was left still in a freezer (-30°C) for 2 hours or more. Then, the vial was removed from the freezer, the opening of the vial was covered with Kimwipes (registered trademark), and the gelatin solution was lyophilized to obtain an intermediate powder containing intermediate particles. The obtained intermediate powder was crosslinked by heating at 150°C for 6 hours to obtain first particles. The synthesized first particles are referred to as "C10MP" or "C10" as appropriate.

<Production of raw material gelatin particles>

Raw material gelatin particles were produced by a method similar to that for the first particles described above, using the raw material gelatin (Org-ApGltn) instead of the synthesized gelatin derivative (C10-ApGltn). The synthesized raw material gelatin particles are referred to as "OrgMP" or "Org" as appropriate.

2. Second particles

<Production of second particles>

By the method described below, $Fe^{2+}$ and $Fe^{3+}$ were coprecipitated under high pH conditions to synthesize second particles (see FIG. 5). The synthesized second particles are referred to as "SPION" or "SP" as appropriate.

(1) $FeCl_2$ (2 g) and $FeCl_3$ (5.4 g) were dissolved in ultrapure water (5 mL) under $N_2$ flow conditions by stirring with a stirrer. (room temperature in draft, rotation speed: 300 rpm).
(2) A 1.8 M aqueous NaOH solution (40 mL) was charged into a 3-necked flask and stirred with a stirrer (1,200 rpm)

while heating to 50°C in an oil bath under $N_2$ flow conditions.

(3) The solution prepared in the above (1) was added dropwise little by little to the aqueous NaOH solution of (2). The dropwise addition produced a black precipitate.

(4) After completion of the dropwise addition, stirring was further continued for 1 hour under the same conditions to obtain a suspension.

(5) The obtained suspension was transferred to a centrifuge tube and centrifuged at 13,000 rpm to remove the supernatant.

(6) 50 mL of ultrapure water was added thereto to resuspend the precipitate to obtain a resuspension. Half of the resuspension was directly ultrasonically washed, and then centrifuged at 13,000 rpm for 60 minutes to remove the supernatant to obtain a precipitate. Further, the precipitate was repeatedly washed nine times by a washing method of adding ultrapure water, centrifuging, and removing the supernatant. Also, the remaining half of the resuspension was washed with ultrasonic waves after adjusting the pH to 7.4, and centrifuged at 13,000 rpm for 60 minutes to remove the supernatant to obtain a precipitate. Furthermore, the precipitate was repeatedly washed six times by the washing method of adding ultrapure water, centrifuging, and removing the supernatant.

When a sufficient amount of precipitate was not obtained by centrifugation, the resuspension was placed on a stirrer overnight to be precipitated by a magnetic field.

(7) The precipitate obtained in the above (6) was dried under reduced pressure to obtain a powder of syn-$Fe_3O_4$ (second particles).

3. Evaluation of particles

The prepared first particles (C10MP), raw material gelatin particles (OrgMP), and second particles (SPION) were observed by SEM. In addition, the particle size distribution and the average particle diameter of each particle were determined. SEM photographs of each particle are shown in FIG. 6, and particle size distributions of each particle are shown in FIGS. 7 and 8. The average particle diameter of each particle was C10MP: 2.3 μm, OrgMP: 3.5 μm, and SPION: 11.4 nm. The conditions for calculating the average particle diameter of each particle are as follows.

<First particles (C10MP), raw material gelatin particles (OrgMP)>

Each particle was observed by scanning electron microscopy (SEM, JCM7000, manufactured by JEOL Ltd., Tokyo, Japan) and particle size was counted by Image J. Specifically, the particle diameter of 100 particles was calculated from the scale length on the SEM image using Image J. The average particle diameter was determined by calculating the average of the particle diameters of 100 particles. For the particle diameter distribution, a frequency distribution table (a table representing the distribution by dividing data by class) was prepared, and the distribution was summarized in a graph.

<Second particles (SPION)>

The average particle diameter of SPION was counted by Image J using images obtained by high-resolution transmission electron microscopy (HRTEM; JEM2100F, manufactured by JEOL Ltd., Tokyo, Japan). Specifically, the particle diameters of 100 particles were randomly calculated from the scale length on the SEM image using Image J. The average particle diameter was determined by calculating the average of the particle diameters of 100 particles. For the particle diameter distribution, a frequency distribution table (a table representing the distribution by dividing data by class) was prepared, and the distribution was summarized in a graph.

4. Powder (1)

<Production of powder>

Samples 1-1 to 1-4, 2-1 to 2-4, and 3 were produced by mixing the produced particles at a mass ratio shown in Table 1.

[Table 1]

| Sample No. | Powder (Mass ratio) | | |
|---|---|---|---|
| | Second Particles (SPION) | Gelatin particles | |
| | | First particles (C10MP) | Raw material gelatin particles (OrgMP) |
| 1-1 | 0 | 50 | - |

(continued)

| Sample No. | Powder (Mass ratio) | | |
|---|---|---|---|
| | Second Particles (SPION) | Gelatin particles | |
| | | First particles (C10MP) | Raw material gelatin particles (OrgMP) |
| 1-2 | 20 | 50 | - |
| 1-3 | 40 | 50 | - |
| 1-4 | 60 | 50 | - |
| 2-1 | 0 | - | 50 |
| 2-2 | 20 | - | 50 |
| 2-3 | 40 | - | 50 |
| 2-4 | 60 | - | 50 |
| 3 | 50 | - | - |

5. Evaluation of powder (1)

<Hydration observation of powder>

Aggregation behavior of the particles in moist environments was observed. Each of Sample 1-3 (SP/C10 = 40 mg/50 mg) and Sample 2-3 (SP/Org = 40 mg/50 mg) was added to 200 μL of physiological saline to prepare a suspension. This suspension was cultured for up to 2 hours, and then the aggregation behavior of the particles was observed using a bright field microscope (BZ-X710, manufactured by KEYENCE CORPORATION). Bright field micrographs are shown in FIG. 9.

In Sample 2-3 (SP/Org), the particles were isolated from each other at any hydration time, and fusion of particles could not be confirmed. On the other hand, in Sample 1-3 (SP/C10), the particles were fused (aggregated) over hydration time, and formed a colloidal gel due to hydrophobic interactions. Sample 1-3 (SP/C10) showed a tightly packed structure after 30 minutes of hydration, and almost all C10MPs were then fused with each other to form large assemblies after hydration for 60 minutes. These results also suggest that hydrophobic interactions between C10MPs are the driving force for formation of a colloidal gel under moist conditions.

In FIG. 9, black portions indicated by white arrows (triangles) are second particles (SP). From this observation, it could be confirmed that in Sample 1-3 (SP/C10), even if the second particles were present, the particles were gelled by hydration without inhibiting hydrophobic interactions between C10MPs, and were fused.

<IR spectrum of powder after hydration (gel)>

The IR spectrum of the gel after hydration was measured for Sample 2-1 (Org), Sample 1-1 (C10), Sample 2-3 (SP/Org), and Sample 1-3 (SP/C10). The results are shown in FIG. 10. For reference, the FT-IR spectrum of Sample 3 is also shown in FIG. 10.

The FT-IR spectrum measurement sample was prepared by the following procedure. First, a predetermined amount of each sample was weighed. Sample 2-1 (Org = 50 mg), Sample 1-1 (C10 = 50 mg), Sample 2-3 (SP/Org = 40 mg/50 mg), and Sample 1-3 (SP/C10 = 40 mg/50 mg). Each of these samples was hydrated with 300 μL of PBS in a silicone mold with a diameter of 10 mm and a thickness of 1.5 mm, the resulting colloidal gel was lyophilized, and a dry colloidal gel was used as a measurement sample.

In FIG. 10(a), focusing on the peak derived from the carboxy group of the gelatin, the peaks of the carboxy groups of Samples 2-1 and 1-1 not containing the second particles are shifted to the low wave number side in Samples 2-3 and 1-3 containing the second particles (1638 $cm^{-1} \rightarrow$ 1629 $cm^{-1}$). From this peak shift, it could be confirmed that in Samples 2-3 and 1-3, the carboxy group contained in the gelatin was coordinate-bonded to the metal (Fe) contained in the second particles (see FIG. 10(b)).

<Storage modulus of powder after hydration (gel)>

Samples 2-1 to 2-4 (using raw material gelatin Org) and Samples 1-1 to 1-4 (using gelatin derivative C10) were evaluated as follows. First, each sample (powder) was weighed at a predetermined mass ratio shown in Table 1 with 50 mg of gelatin particles (Org or C10). Each of these samples was placed in a silicone mold with a diameter of 10 mm and a

thickness of 1.5 mm, and then the surface of the powder was smoothed using a spatula. Thereafter, 300 μL of PBS was placed on each sample (powder) and hydrated at 37°C for 30 minutes to obtain a measurement sample. The prepared sample for measurement was placed on a stage of a viscoelasticity measuring apparatus (Rheoplus, Anton Paar) and sandwiched between jigs with a diameter of 10 mm, and the storage modulus G' of the sample was measured under the conditions of a stage temperature of 37°C, a shear strain of 1%, and an angular frequency of 10 rad/s. The results are shown in FIG. 11.

As shown in FIG. 11, Samples 1-1 to 1-4 (using gelatin derivative C10) had a high storage modulus G' and a gel with higher strength was formed as compared to Samples 2-1 to 2-4 (using raw material gelatin Org). As confirmed in <Hydration observation of powder> (see FIG. 9), it is inferred that the particles were fused (aggregated) due to hydrophobic interaction between the hydrophobic groups (decyl groups) introduced into the gelatin derivative of the first particles, thereby increasing the gel strength (see FIG. 12).

In addition, in FIG. 11, comparing Samples 1-1 to 1-4, the higher the proportion of the contained second particles (SP), the higher the gel strength. As confirmed in <IR spectrum after hydration of powder> (see FIG. 10), it is inferred that the gel strength was further enhanced by the interaction between the second particles and the carboxy group of the gelatin derivative (see FIG. 12).

As described above, in Samples 1-1 to 1-4, as shown in FIG. 12, it is inferred that two types of interactions, the interaction between the first particles (C10MP-C10MP interaction) and the interaction between the first particles and the second particles (SPION-C10MP interaction) contribute to the improvement of the gel strength.

<Adhesion strength>

Samples 2-1 to 2-4 (using raw material gelatin Org) and Samples 1-1 to 1-4 (using gelatin derivative C10) were evaluated as follows. The test method was performed according to the American Society for Testing and Materials (ASTM F-2258-05) (see FIG. 13).

First, fresh porcine stomach (purchased from Tokyo Shibaura Zouki) was opened, and the mucosa layer was removed to expose a serosa tissue which is a submucosal tissue. The resulting tissue was cut into tissue pieces of 2.5 cm square, and fixed on each stage of the upper and lower jigs of a test apparatus (a texture analyzer TA-XT2i, manufactured by Stable Micro Systems) using a cyanoacrylate glue (manufactured by Henkel Japan Ltd.). A hot plate was used to maintain the temperature of the porcine stomach lining tissue at 37°C during the measurements. In order to remove excess surface water on the above tissue, an industrial paper rag (trade name "Kimwipe") was pressed against the surfaces at 80 kPa for 3 minutes to remove water (FIG. 13(a)). Each sample was sprinkled so as to cover the tissue on the stage of the lower jig (FIG. 13(b)). Thereafter, the upper jig was placed over the sample on the lower jig, and crimped under a pressure of 80 kPa for 3 minutes (FIG. 13(c)). The upper jig was then lifted up at 10 mm/min, and the adhesion strength of the sample was measured (FIG. 13(d)). The results are shown in FIG. 14.

As a control, the adhesion strength between two tissues over which the particles were not sprinkled is also shown in FIG. 14 (No materials). The amount of each sample used in the test (the amount sprinkled on the tissue) was an amount at a predetermined mass ratio shown in Table 1 when 50 mg of gelatin particles (Org or C10) was used.

Further, for comparison, the results of tests performed in the same manner using 20 mg, 40 mg, and 60 mg of SP (second particles) instead of each sample are also shown in FIG. 14.

As shown in FIG. 14, Samples 1-1 to 1-4 (using gelatin derivative C10) had high adhesion strength as compared to Samples 2-1 to 2-4 (using raw material gelatin Org). This is inferred to be because, as shown in FIG. 3, hydrophobic interaction between the first particles 10, hydrophobic interaction between the first particles 10 and the tissue (cell membrane, cell matrix), and interaction between the second particles and the first particles (for example, a coordinate bond between metal-containing second particles and carboxy groups in the first particles) occurred.

Next, Samples 1-1 to 1-4 are compared in FIG. 14. As the proportion of the contained second particles (SP) increased, the adhesion strength increased, reaching a peak at SP/C10 = 40/50, and when the proportion of the second particles further increased, the adhesion strength slightly decreased. The reason why the adhesion strength increases as the proportion of the second particles (SP) increases is inferred to be that the gel strength increases due to the interaction between the first particles and the second particles. In addition, the reason why the gel strength slightly decreased in Sample 1-4 (SP/C10 = 60/50) is inferred to be that the mass ratio of the second particles (SP) exceeded that of the first particles, thus affecting the interaction between the first particles and the tissue (weakening the interaction).

<Pressure resistance>

Using Samples 2-1 and 2-3 (using raw material gelatin Org) and Samples 1-1 and 1-3 (using gelatin derivative C10), the pressure resistance of the colloidal gel was measured by a method in which the conditions were finely modified based on the method of ASTM-F2392-04R (see FIG. 15). First, the mucosal tissue of the duodenum was cut into a circle with a diameter of 35 mm, the submucosal layer with a diameter of 10 mm was removed with surgical scissors to expose the

submucosal tissue, and a 1 mm pinhole was formed at the center of the exposed submucosal tissue to create a perforation model after ex-vivo ESD (endoscopic submucosal dissection) (FIG. 15(a)). In the perforation model, each sample (powder) was applied using a silicone mold to a region with a diameter of 10 mm and a thickness of 1.5 mm so as to close the pinhole (FIG. 15(b)), and 300 μL of PBS was added dropwise to hydrate the sample (FIG. 15(c)). Thereafter, the sample was left for 30 minutes to form a colloidal gel. The tissue in which the colloidal gel was formed was fixed to a pressure resistance tester, and air pressure was applied from the gel side at a rate of 2 mL/min to measure the pressure resistance of the colloidal gel (FIG. 15(d)).

The amount of each sample used in the test is as follows. Sample 2-1 (Org = 50 mg), Sample 2-3 (SP/Org = 40 mg/50 mg), Sample 1-1 (C10 = 50 mg), and Sample 1-3 (SP/C10 = 40 mg/50 mg).

As shown in FIG. 16, Samples 1-1 and 1-3 (using gelatin derivative C10) had high pressure resistance as compared to Samples 2-1 and 2-3 (using raw material gelatin Org). Similarly to the results of <Adhesion strength test>, this is inferred to be because hydrophobic interaction between the first particles 10, hydrophobic interaction between the first particles 10 and the tissue (cell membrane, cell matrix), and interaction between the second particles and the first particles (for example, a coordinate bond between metal-containing second particles and carboxy groups in the first particles) occurred (see FIG. 3).

Also, in FIG. 16, comparing Sample 1-1 and Sample 1-3, Sample 1-3 in which the proportion of the contained second particles (SP) was high tended to have a slightly higher pressure resistance. This is inferred to be because the gel strength was increased by the interaction between the first particles and the second particles.

<Heat generation test of powder after hydration (gel)>

For the gels obtained from Samples 1-1 to 1-4 (powders), the heat generation characteristics in response to an alternating magnetic field (AMF) were measured using a magnetic field generator (G2 D5, Nanoscale Biomagnetics, Zaragoza, Spain). First, each sample (powder) was placed in a silicone mold with a diameter of 10 mm and a thickness of 1.5 mm, and hydrated with 300 μL of PBS to prepare a colloidal gel. The resulting colloidal gel was placed on a stage of a magnetic field generator, and the colloidal gel was loaded with 130 G, 373.35 kHz AMF for up to 600 seconds. The temperature change on the colloidal gel was measured by a thermal camera (Xi 400, Optris, Berlin, Germany). The results are shown in FIG. 17.

The amount of each sample used in the test was set to an amount at a predetermined mass ratio shown in Table 1 with the gelatin particles (C10) as 50 mg.

As shown in FIG. 17, the higher the ratio of SP in the sample, the more the heat generation characteristics were also improved. In particular, in Sample 1-3 (SP/C10 = 40/50) and Sample 1-4 (SP/C10 = 60/50), the temperature after application of the magnetic field was 40°C or higher, which is a temperature sufficient to kill cancer cells.

<Test using powder as cancer killing material (in vitro)>

In order to confirm the effect of Sample 1-3 (SP/C10 = 40/50) as a cancer killing material, tests (A-1) to (A-4) and tests (B-1) to (B-4) described below were performed.

Test (A-1):

First, KM12-Luc cells as cancer cells were cultured in DMEM medium containing 10% FBS and 1% penicillin-streptomycin. KM12-Luc cells were seeded at a concentration of $3.0 \times 10^5$ cells/well in 8-well glass plates and incubated at 37°C for 24 hours in a 5% $CO_2$ atmosphere. In addition, using a silicone mold of 8 mm × 8 mm × depth 1 mm, a gel (colloidal gel) was prepared by hydrating Sample 1-3 (SP/C10 = 40 mg/50 mg) in the mold with 300 μL of PBS. The resulting colloidal gel was added to incubated KM12-Luc cells and the colloidal gel was loaded with AMF (130 G, 373.35 kHz) for 10 minutes. After the colloidal gel was removed from the medium, the medium was replaced with 10% WST-8/DMEM and incubated for 2 hours. Finally, the absorbance at 450 nm was measured by WST-8 assay and the number of cells was counted from the calibration curve. The results are shown in FIG. 18.

Test (A-2): A test was performed in the same manner as in the test (A-1) except that Sample 1-3 (SP/C10) was not used.

Test (A-3): A test was performed in the same manner as in the test (A-1) except that the colloidal gel was not loaded with AMF.

Test (A-4): A test was performed in the same manner as in the test (A-1) except that Sample 1-3 (SP/C10) was not used and the colloidal gel was not loaded with AMF.

The results of the above tests (A-2) to (A-4) are shown in FIG. 18.

Tests (B-1) to (B-4):

A test was performed in the same manner as in each of the tests (A-1) to (A-4) except that hMSCs (human mesenchymal stem cells), which are normal cells, were used instead of the cancer cells. The results are shown in FIG. 19.

As shown in FIG. 18, it could be confirmed that a high cancer cell killing effect can be obtained by loading Sample 1-3 (SP/C10) (Test A-1) with AFM. This is inferred to be because Sample 1-3 was heated to 40°C or higher by being loaded with AFM (see FIG. 17).

On the other hand, as shown in FIG. 19, when Sample 1-3 (SP/C10) was loaded with AFM on normal cells (hMSCs) (Test B-1), cell killing was observed, but the decrease in the number of cells was not as large as that of cancer cells. This is because the normal cells are resistant to high-temperature environments as compared to the cancer cells.

From the above results, it could be confirmed that Sample 1-3 (SP/C10) functions as a cancer killing material having a small damage to the normal cells by being loaded with AFM.

<Test using powder as cancer killing material (in vivo) (1)>

In order to confirm the effect of Sample 1-3 (SP/C10 = 40/50) as a cancer killing material, the test described below was performed.

First, a PBS solution containing cultured cancer cells (KM12-Luc cells) at a concentration of $2.5 \times 10^6$ cells/0.1 mL was injected into the subcutaneous tissue of the back of a nude mouse. A mouse in which after injection of cancer cells, the cancer cells proliferated and the size of the cancer (tumor volume) increased to 100 $mm^3$ was used as a tumor-bearing mouse model in the following test.

The size of the cancer (tumor volume) in mouse was determined by the following method. First, 30 mg/mL of luciferin (100 μL) was injected into the abdomen of the mouse, and after 10 minutes, luminescence of the cancer was confirmed using IVIS imaging system (Lumina 2, PerkinElmer Inc., Waltham, MA, USA.). From the luminescence, a: length of the cancer, b: width of the cancer were calculated, and the size of the cancer was calculated according to the following formula.

$$\text{Size of cancer (tumor volume) (mm}^3\text{)} = (ab^2)/2$$

In the above formula, a represents the length of the cancer (the length at the longest position of the tumor), and b represents the width of the cancer (the length at the shortest position of the tumor).

The mice were divided into two groups, Group (I) (n = 5), Group (II) (n = 5), and both groups created a subcutaneous space between the cancer and the abdominal tissue after anesthesia. A gel (diameter 10 mm, thickness 1.5 mm) prepared from Sample 1-3 (SP/C10) was transplanted only into the subcutaneous space of mice in Group (I). The transplanted gel was prepared by hydrating Sample 1-3 (SP/C10=40 mg/50 mg) with 300 μL of PBS.

The mice in Groups (I) and (II) were loaded with AMF (130 G and 373.35 kHz) daily for Days 1 to 3 and every two days after Day 4 and the temperature of the skin surface was monitored with a thermo camera. Similarly, the size of the cancer was determined from the luminescence of the cancer by the IVIS observation described above daily for Days 1 to 3 and every two days after Day 4. These AMF loading and IVIS observations were repeated for 12 days. Changes in the size of cancer (tumor volume) in mice in Groups (I) and (II) are shown in FIG. 20.

In addition, AMF loading and IVIS observations were also performed every two days after Day 12 to calculate the size of cancer (tumor volume) in mice in Groups (I) and (II), and when the length a of the cancer reached 20 mm, the mice were sacrificed as a humane endpoint, and the survival rate was calculated. After 18 days, all mice were sacrificed. The survival rates of mice in Groups (I) and (II) are shown in FIG. 21.

As shown in FIG. 20, in Group (II) (without gel transplantation), cancer continued to grow from the start of the test until Day 12. On the other hand, in Group (I) (with SP/C10 gel transplantation), the proliferation of cancer could be greatly suppressed. Also, as shown in FIG. 21, in Group (II) (without gel transplantation), the mouse survival rate was 20% on Day 16 from the start of the test. On the other hand, in Group (I) (with SP/C10 gel transplantation), the mouse survival rate on Day 16 was 100%.

6. Powder (2) (Powder forming colloidal gel having tissue adhesion/sustained release ability of anticancer agent)

<Production of powder>

As the first particles, C10MP was used. The details of the production method are as described above. In addition,

the average particle diameter of the first particles is 2.3 μm, and the conditions for calculating the average particle diameter of the first particles are also as described above.

Paclitaxel (PTX) reprecipitated with commercially available paclitaxel (PTX) (manufactured by Nacalai Tesque, Inc.) was used as the second particles. The reprecipitation was performed in the following procedure.

(1) Paclitaxel (PTX; 20 mg) was dissolved in 2 mL of ethanol at 25°C to prepare a 1w/v% PTX solution.
(2) While stirring at 400 rpm, 2 mL of ultrapure water at 25°C as a poor solvent was added dropwise thereto, and PTX was recrystallized in the solution.
(3) The recrystallized PTX was pre-frozen at -30°C for 24 hrs.
(4) PTX crystals were obtained by lyophilizing for 24 hrs.

In addition, the crystal structures of commercial paclitaxel (PTX) and reprecipitated paclitaxel (PTX) were analyzed by XRD. The analysis by XRD was performed in the following procedure: the commercial PTX and the reprecipitated PTX were fixed in a silicon sample holder. The sample holder was set in an XRD device (Powder XRD/SmartLab3, Rigaku), and measurement was performed under the following conditions.

[Table 2]

| **Measurement Conditions** | |
|---|---|
| 2θ | 5°<2θ<60° |
| Speed | 3°/min |
| Incident slit | 1°/2 |
| Receiving slit 1 | 20.00mm |
| Receiving slit 2 | Open |

FIG. 23 shows the X-ray diffraction patterns of commercial PTX and reprecipitated PTX. As shown in FIG. 23, since the peak position of reprecipitated PTX coincided with the peak position observed in commercial PTX, it was confirmed that the reprecipitated PTX had a crystal structure. On the other hand, the crystallinity of commercial PTX was 74 ± 3%, whereas the crystallinity of reprecipitated PTX was 40 ± 3%. Therefore, the crystallinity can be reduced by reprecipitation, and it is predicted that the elution speed of PTX from the gel increases due to the reduction in crystallinity.

Also, the average particle diameter of the second particles was 2.8 μm. The conditions for calculating the average particle diameter are the same as the conditions for calculating the average particle diameter of the second particles described above.

As shown in FIG. 22, the first particles 10 and the second particles 50 were mixed at a mass ratio (first particles/second particles) of 50/1 to produce a powder 60 composed of C10MP and paclitaxel (PTX) (referred to as PTX/C10MP as appropriate) as Sample 4.

<Water contact angle of powder (2)>

The powder (PTX: 0.4 mg/C10MP: 20 mg) produced by the procedure described above was weighed into a 2 mL tube and stirred with a vortex for 10 seconds. The mixed particles were fixed on a glass slide with a double-sided tape (range 1 × 2 $cm^2$). A water contact angle one second after dropping 10 μL of water droplets on the particles from a contact angle measuring device (DM700, Kyowa Interface Science Co., Ltd) was measured. The water contact angle was similarly measured for OrgMP, C10MP, and PTX/OrgMP as comparison objects. The measurement results are shown in FIGS. 24(a) and 24(b). FIG. 24(a) shows photographs of the shape of a water droplet on each particle or powder, and FIG. 24(b) shows the water contact angles of each particle or powder. In the hydrophobized gelatin particles or the powder containing the particles, the water contact angle increased, whereas there was no change in the water contact angle depending on the presence or absence of PTX.

<Water content of colloidal gel formed from PTX/C10MP>

The powder (PTX: 1 mg/C10MP: 250 mg) produced by the above-described procedure was weighed into a 2 mL tube, and stirred with a vortex for 10 seconds. The powder was added to a silicone mold (diameter (D): 10 mm × depth: 1 mm) and hydrated with 500 μl of PBS for 30 min. Excess PBS was removed with Kimwipes before measuring the mass of the gel (WW). After lyophilizing the sample, the dry gel mass (WD) was measured. The water content was calculated from the following formula.

$$\text{Water content (\%)} = 100 \times (\text{WW - WD})/\text{WW}$$

WW: Colloidal gel weight in wet state
WD: Colloidal gel weight in dry state

The water content was similarly calculated for gels formed from OrgMP, C10MP, and PTX/OrgMP (PTX: 1 mg/OrgMP: 250 mg) as comparison objects. FIG. 25 shows the measurement results. As shown in FIG. 25, in the gels formed from C10MP, the gel water content formed from OrgMP was reduced. This is considered to be because the crosslinking density in the gel was increased by hydrophobic interaction. Such a structure is expected to improve pressure resistance.

<Pressure resistance of PTX/C10 colloidal gel>

The pressure resistance of the colloidal gel was measured by a method in which the conditions were finely modified based on the method of ASTM-F2392-04R. As shown in FIG. 26, the porcine gastric mucosa was washed, physiological saline was injected into the submucosal layer tissue with a needle, the mucosa was locally injected, and then the mucosal tissue was excised with scissors. Subsequently, the mucosal tissue was cut into a circle with a diameter of 35 mm, and a pinhole with a diameter of 3 mm was created at the center of the circle to create a perforation model after ex-vivo ESD (endoscopic submucosal dissection).

PTX/OrgMP (PTX: 1 mg/OrgMP: 50 mg) or PTX/C10MP (PTX: 1 mg/C10MP: 50 mg) was sprayed to a region with a diameter of 15 mm at a thickness of 1 mm using a silicone mold so as to close the pinhole. After the particles were left on the tissue for 10 minutes, 500 μL of PBS was added onto the particles and left for 30 minutes to form a colloidal gel.

The tissue in which the gel was formed was fixed to a pressure resistance tester, air pressure was applied from the gel side, the pressure at which the gel was broken was measured, and the pressure resistance was evaluated.

FIG. 27(a) shows the measurement results, and FIG. 27(b) schematically shows the internal structure of the formed colloidal gel. As shown in FIG. 27(a), the pressure resistance was significantly increased by hydrophobization. In addition, the pressure resistance was further increased by mixing the PTX powder with C10MPs. This is considered to be because the hydrophobic PTX interacts with the hydrophobic group of the gelatin, and the PTX acts as a composite material. Similar effects are expected for other hydrophobic compounds.

<PTX sustained release characteristics of PTX/C10MP colloidal gel>

50 mg OrgMP or C10-MP and 1.0 mg paclitaxel were weighed into a 2 mL tube and stirred with a vortex for 10 seconds to obtain a powder. In addition, 1.0 mg of paclitaxel was used alone as a comparison object.

PTX/OrgMP, PTXC10MP or PTX was placed in a silicone mold (diameter (D) = 10 mm), PBS (500 μL) was added dropwise thereto, and the mixture was hydrated for 30 minutes to form a gel.

The formed colloidal gel was immersed in PBS (50 mL) in a 50 mL centrifuge tube, incubated at 37°C, the tube was inverted and mixed 3 times on the passage of 3, 6, 9, 12, 15, and 18 days, and the supernatant (10 mL) was collected for measurement and stored at -30°C.

After complete removal of the solution on the gel, fresh PBS (50 mL) was added and incubated again at 37°C.

The collected sample was lyophilized and then dissolved in the following eluent.

The concentration of PTX eluted by HPLC was calculated using the obtained sample. The measurement conditions are as follows.

Eluent: acetonitrile/water/methanol = 48/41/11 (v/v)

Flow rate: 1 mL/min
UV wavelength: 242 nm
Column: C18 Nova-Pak column (Waters)

FIG. 28 shows the measurement results. Since PTX, PTX/Org were not stable in PBS, most of PTX diffused into PBS within 9 days. On the other hand, it was revealed that PTX/C10 continuously releases PTX slowly for 18 days, and releases 37% of all PTX slowly for 18 days.

<Test using powder as cancer killing material (in vivo) (2)>

As shown in FIG. 29, 100 μL each of PBS in which human large bowel cancer-derived cells (KM12 Luc) were suspended ($2.5 \times 10^7$ cells/mL) was injected into the back of BALB/c nude mice, and 8 days later, it was confirmed that the average tumor volume was 75 $mm^3$.

The following gels or suspensions were then implanted or injected into the tissue between the tumor and peritoneum of the mice.

Group (I) (n = 5): control (untreated)
Group (II) (n = 3): PTX (1 mg/mL PTX/PBS suspension)
Group (III) (n = 3): OrgMP (50 mg) gel
Group (IV) (n = 3): C10MP (50 mg) gel
Group (V) (n = 3): PTX/OrgMP (1 mg/50 mg) gel
Group (VI) (n = 3): PTX/C10MP (1 mg/50 mg) gel

The mice were observed every three days, and the tumor volume was determined according to the following calculation formula.

$$\text{Tumor volume (mm}^3\text{)} = (ab^2)/2$$

wherein a represents the length of the cancer (the length at the longest position of the tumor), and b represents the width of the cancer (the length at the shortest position of the tumor). In addition, tumor imaging by IVIS was performed every six days. IVIS was observed 10 minutes after injection of 100 μL of 30 mg/mL luciferin/PBS into the peritoneum of the mice.

FIG. 30 shows temporal changes in tumor volume of each group, and FIG. 31 shows temporal changes in survival rate of each group. As shown in FIG. 30, the PTX/C10MP group significantly suppressed tumor growth on Day 12 as compared to other groups.

INDUSTRIAL APPLICABILITY

The powder of the present embodiment can be used for a tissue covering material having various functions. Examples of the tissue covering material include a cancer killing material and a bone filling material, and the tissue covering material can also be used as a wound dressing, an anti-adhesion material, a hemostatic material, or the like.

REFERENCE SIGNS LIST

| | |
|---|---|
| 10 | First particles |
| 20, 50 | Second particles |
| 30, 60 | Powder(s) |
| 40, 70 | Gel(s) |

**Claims**

**1.**

A powder comprising first particles containing a crosslinked gelatin derivative; and second particles which are different from the first particles and can provide a further function,
wherein the gelatin derivative has a structure represented by the following formula (1):

[Chemical Formula 1] **GtlnNH-L-CHR$^1$R$^2$** • • • (1)

wherein Gltn represents a residue of gelatin, L represents a single bond or a divalent linking group, $R^1$ represents a hydrocarbon group having 1 to 20 carbon atoms, and $R^2$ represents a hydrogen atom or a hydrocarbon group having 1 to 20 carbon atoms.

**2.** The powder according to claim 1, wherein the second particles contain a particulate pharmaceutical agent.

**3.** The powder according to claim 2, wherein the pharmaceutical agent is hydrophobic or positively or negatively charged.

**4.** The powder according to any one of claims 1 to 3, wherein a ratio of a mass of the second particles (W2) to a mass of the first particles (W1) (W2/W1) is 20/50 to 60/50.

**5.** The powder according to claim 4, wherein the ratio of a mass of the second particles (W2) to a mass of the first particles (W1) (W2/W1) is 0.1/50 to 40/50.

**6.** The powder according to claim 4, wherein the ratio of a mass of the second particles (W2) to a mass of the first particles (W1) (W2/W1) is 1/50 to 60/50.

**7.** The powder according to any one of claims 1 to 6, wherein an average particle diameter of the first particles is 0.1 μm to 100 μm.

**8.** The powder according to any one of claims 1 to 7, wherein an average particle diameter of the second particles is 5 nm to 100 μm.

**9.** The powder according to claim 8, wherein the average particle diameter of the second particles is 10 nm to 100 nm.

**10.** The powder according to any one of claims 1 to 9, wherein a ratio of the average particle diameter of the second particles to the average particle diameter of the first particles (D2/D1) is 0.001 to 0.1.

**11.** The powder according to any one of claims 1 to 10, wherein the powder has a water contact angle of 50 degrees or more as the water contact angle was measured one second after water is added dropwise.

**12.** The powder according to any one of claims 2 to 11, wherein the pharmaceutical agent is at least one selected from the group consisting of an anticancer agent, an immunosuppressant, a growth factor, a cell differentiation inducer, an antimetabolite, a growth factor inhibitor, an immune checkpoint inhibitor, an antibiotic, an anti-inflammatory agent, and a protein preparation.

**13.** The powder according to claim 12, wherein the pharmaceutical agent contains at least one selected from the group consisting of a hydrophobic anticancer agent, a hydrophobic immunosuppressant, and a hydrophobic cell differentiation inducer.

**14.** The powder according to claim 13, wherein the crystallinity of the hydrophobic anticancer agent is 30 to 50% as determined by XRD crystallographic analysis.

**15.** The powder according to claim 13 or 14, wherein the hydrophobic anticancer agent is selected from the group consisting of cabazitaxel, paclitaxel, docetaxel, cisplatin, carboplatin, nedaplatin, oxaliplatin, tamoxifen, anastrozole, glivec, 5-fluorouracil (5-FU), floxuridine, leuprolide, flutamide, zoledronate, doxorubicin, vincristine, gemcitabine, streptozocin, vinorelbine, retinoic acid series, adriamycin, mitomycin, daunomycin, prednisone, testosterone, mitoxantrone, aspirin, salicylic acid, ibuprofen, naproxen, fenoprofen, indomethacin, phenyltazone, cyclophosphamide, celecoxib, valdecoxib, and nimesulide.

**16.** The powder according to claim 14, wherein the hydrophobic immunosuppressant is selected from the group consisting of steroids (for example, hydrocortisone, cortisone acetate, prednisone, prednisolone, methylprednisolone, triamcinolone, dexamethasone, betamethasone), cyclosporine, mizoribine (bredinin), cyclophosphamide (endoxan), azathioprine (azanin), tacrolimus (FK506), sirolimus (rapamycin), deoxyspergualin (gusperimus hydrochloride), everolimus, ABT-578 (zotarolimus), basiliximab (an anti-IL-2 receptor monoclonal antibody), muromonab CD3 (an anti-CD3 monoclonal antibody), and arbulin (an anti-lymphocyte globulin).

**17.** The powder according to claim 14, wherein the hydrophobic cell differentiation inducer is tamibarotene.

**18.** The powder according to any one of claims 2 to 13, wherein the pharmaceutical agent contains a growth factor and the powder further contains a cationic polymer.

**19.** The powder according to claim 18, wherein the growth factor is selected from the group consisting of vascular

endothelial growth factor (VEGF), basic fibroblast growth factor (bFGF), platelet-derived growth factor (PDGF), hepatocyte growth factor (HGF), transforming growth factor-β (TGF-β), and Wnt7b.

**20.** The powder according to any one of claims 1 to 19, wherein the second particles contain inorganic fine particles.

**21.** The powder according to claim 20, wherein the second particles contain magnetic particles and an anticancer agent, preferably a hydrophobic anticancer agent.

**22.** The powder according to claim 20, wherein the second particles contain calcium phosphate and a growth factor.

**23.** A tissue covering material comprising the powder according to any one of claims 1 to 22.

**24.** A cancer killing material comprising the powder according to claim 21.

**25.** The cancer killing material according to claim 24, wherein the magnetic particles are iron oxide.

**26.** A bone filling material comprising the powder according to claim 22.

**27.** The powder according to claim 1, wherein the second particles are inorganic fine particles.

**28.** The powder according to claim 27, wherein the second particles contain a metal.

**29.** The powder according to claim 27 or 28, wherein a ratio of a mass of the second particles (W2) to a mass of the first particles (W1) (W2/W1) is 20/50 to 60/50.

**30.** The powder according to claim 29, wherein the ratio of a mass of the second particles (W2) to a mass of the first particles (W1) (W2/W1) is 20/50 to 40/50.

**31.** The powder according to claim 29, wherein the ratio of a mass of the second particles (W2) to a mass of the first particles (W1) (W2/W1) is 40/50 to 60/50.

**32.** The powder according to any one of claims 27 to 31, wherein an average particle diameter of the first particles is 0.1 μm to 100 μm.

**33.** The powder according to any one of claims 27 to 32, wherein an average particle diameter of the second particles is 5 nm to 100 μm.

**34.** The powder according to claim 33, wherein the average particle diameter of the second particles is 10 nm to 100 nm.

**35.** The powder according to any one of claims 27 to 34, wherein a ratio of the average particle diameter of the second particles to the average particle diameter of the first particles (D2/D1) is 0.001 to 0.1.

**36.** The powder according to any one of claims 27 to 35, wherein the second particles are magnetic particles or calcium phosphate.

**37.** The powder according to any one of claims 27 to 36, wherein the powder has a water contact angle of 50 degrees or more as the water contact angle was measured one second after water is added dropwise.

**38.** A tissue covering material comprising the powder according to any one of claims 27 to 37.

**39.** A cancer killing material comprising the powder according to claim 36, wherein the second particles are magnetic particles.

**40.** The cancer killing material according to claim 39, wherein the magnetic particles are iron oxide.

**41.** A bone filling material comprising the powder according to claim 36, wherein the second particles are calcium phosphate.

**42.** A method for covering or filling a tissue, comprising topically administering an effective amount of the powder according to any one of claims 1 to 22 and 27 to 37 to a predetermined site of the tissue to form a gel.

**43.** A method for covering or filling an excised site of a tissue, comprising the step of topically administering an effective amount of the powder according to any one of claims 1 to 22 and 27 to 37 to an excised site of a patient after surgery with tissue excision or its periphery to form a gel.

**44.** The method according to claim 43, wherein the second particles contain an anticancer agent, and the tissue is tumor tissue.

**45.** A method for treating cancer in a patient with a tumor, comprising the step of topically administering a therapeutically effective amount of the powder according to any one of claims 12 to 15, 20, and 21 to a tumor tissue or its periphery in the patient to form a gel, wherein the second particles contain an anticancer agent.

**46.** The method according to claim 45, wherein the second particles further contain magnetic particles.

**47.** A method for treating or alleviating ulcerative colitis in a patient suffering from ulcerative colitis, comprising the step of topically administering a therapeutically effective amount of the powder according to any one of claims 12, 13, and 16 to an inflamed site or its periphery in the patient to form a gel, wherein the second particles contain an immunosuppressant.

**48.** A method for treating polycystic kidney disease in a patient with autosomal dominant polycystic kidney disease, comprising the step of topically administering a therapeutically effective amount of the powder according to any one of claims 12, 13, and 17 to a renal tissue or its periphery in the patient to form a gel, wherein the second particles contain a cell differentiation inducer.

**49.** A method for promoting tissue regeneration in a patient after tissue removal, comprising the step of topically administering a therapeutically effective amount of the powder according to any one of claims 12, 13, and 18 to a tissue removal site or its periphery in the patient to form a gel, wherein the second particles contain a growth factor.

**50.** A method for killing residual malignant pleural mesothelioma in a patient after removal of malignant pleural mesothelioma, comprising topically administering a therapeutically effective amount of the powder according to claim 12 to a cancer removal site or its periphery in the patient to form a gel, wherein the second particles contain an antimetabolite.

**51.** A method for treating malignant pleural mesothelioma in a patient with malignant pleural mesothelioma, comprising the step of topically administering a therapeutically effective amount of the powder according to claim 12 to a cancer tissue or a cancer tissue removal site or its periphery in the patient to form a gel, wherein the second particles contain a growth factor inhibitor.

**52.** A method for treating kidney cancer, non-small cell lung cancer, head and neck cancer, melanoma, Hodgkin's disease, or liver cancer in a patient with kidney cancer, non-small cell lung cancer, head and neck cancer, melanoma, Hodgkin's disease, or liver cancer, comprising the step of topically administering a therapeutically effective amount of the powder according to claim 12 to a cancer tissue or a cancer removal site tissue or its periphery in the patient to form a gel, wherein the second particles contain an immune checkpoint inhibitor.

**53.** The method according to any one of claims 42 to 52, wherein the gel has a water content of 50 to 70%.

**54.** The method according to any one of claims 42 to 53, wherein the gel has a pressure resistance of 70 mmHg or more.

FIG. 1

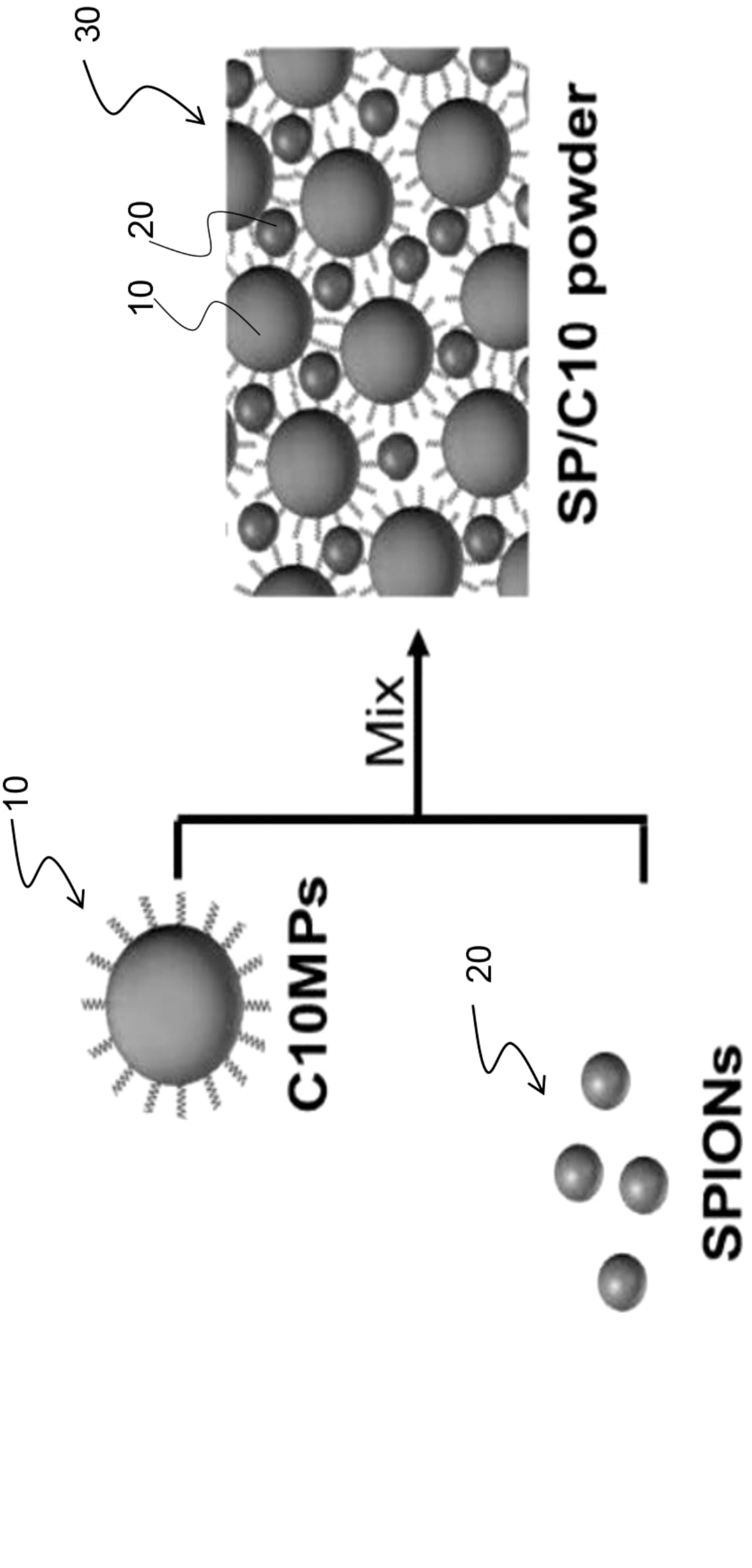

FIG. 2

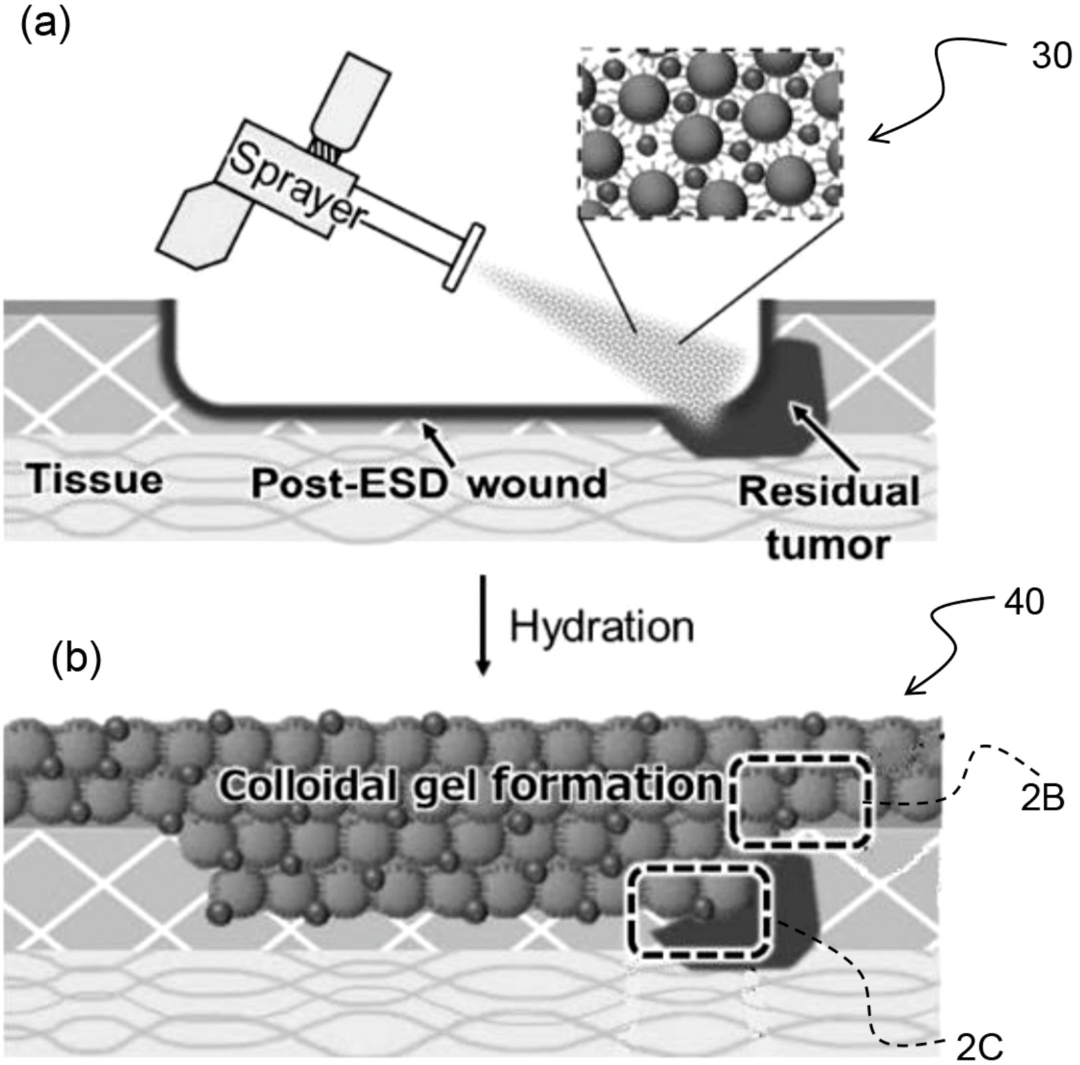
(a)
30
Sprayer
Tissue
Post-ESD wound
Residual
tumor
Hydration
(b)
40
Colloidal gel formation
2B
2C

FIG. 3

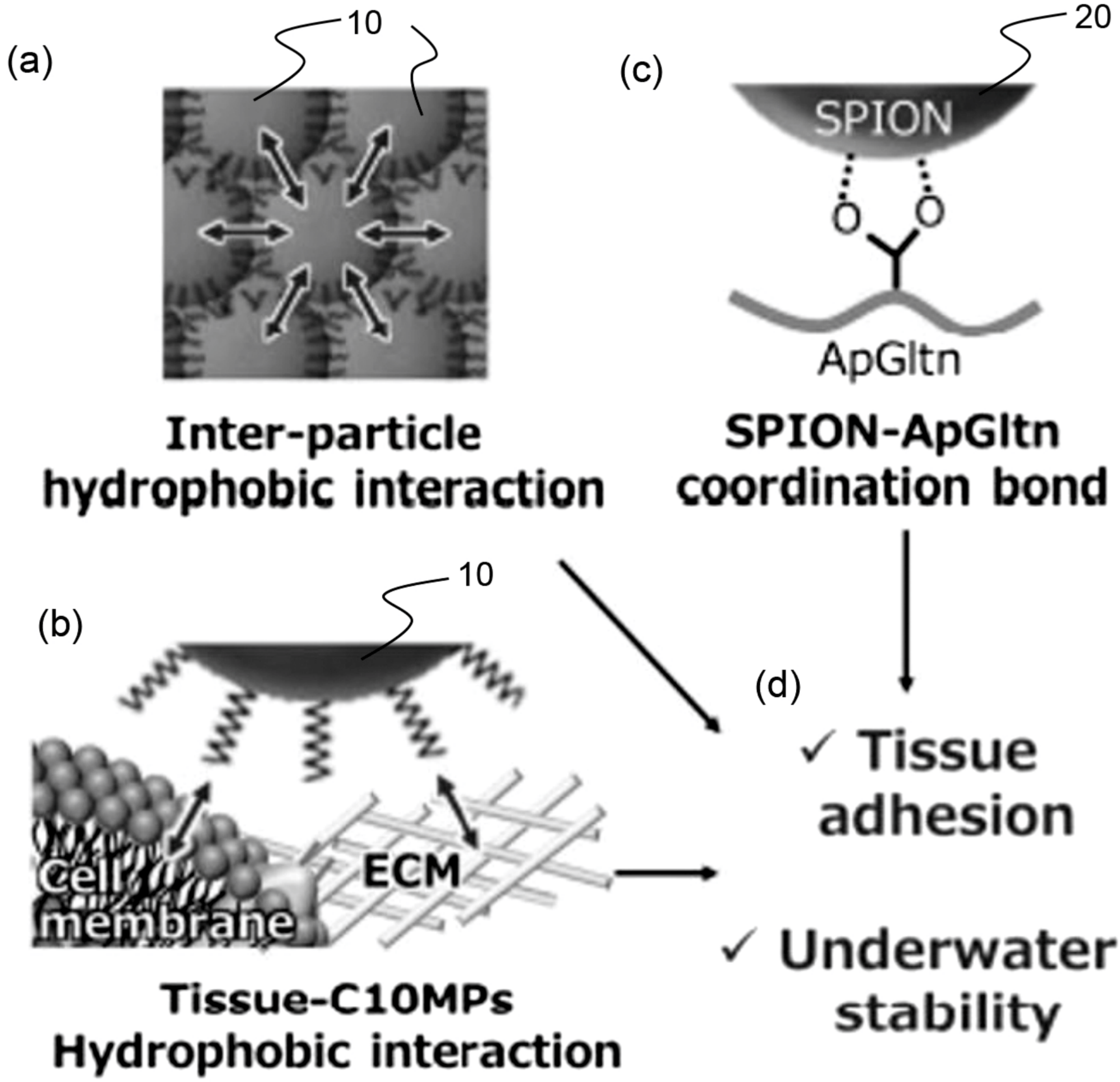
(a)
10
Inter-particle
hydrophobic interaction
(c)
20
SPION
O
O
ApGltn
SPION-ApGltn
coordination bond
(b)
10
Cell
membrane
ECM
Tissue-C10MPs
Hydrophobic interaction
(d)
✓ Tissue
adhesion
✓ Underwater
stability

FIG. 4

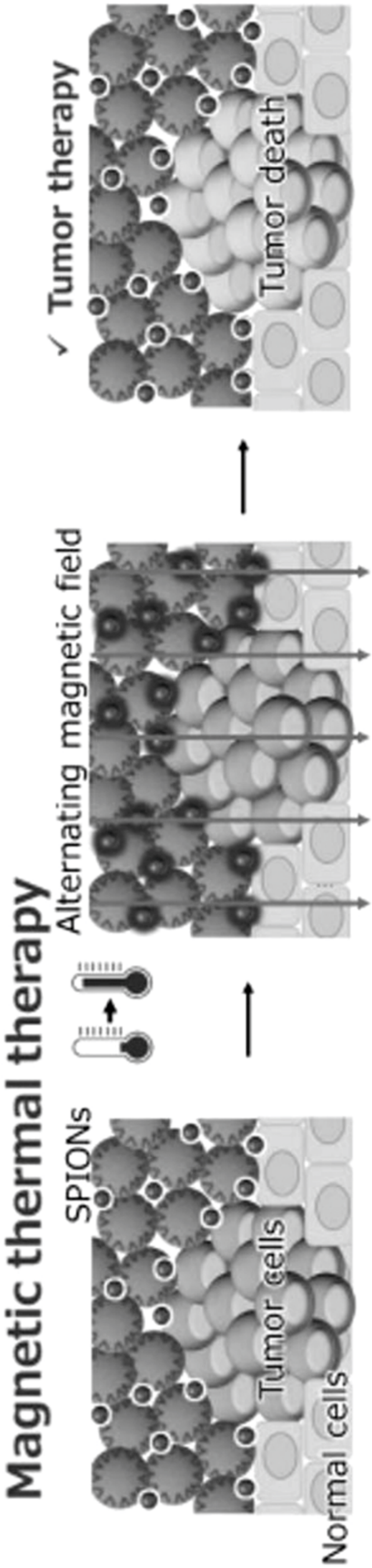
Magnetic thermal therapy
SPIONs
Tumor cells
Normal cells
Alternating magnetic field
✓ Tumor therapy
Tumor death

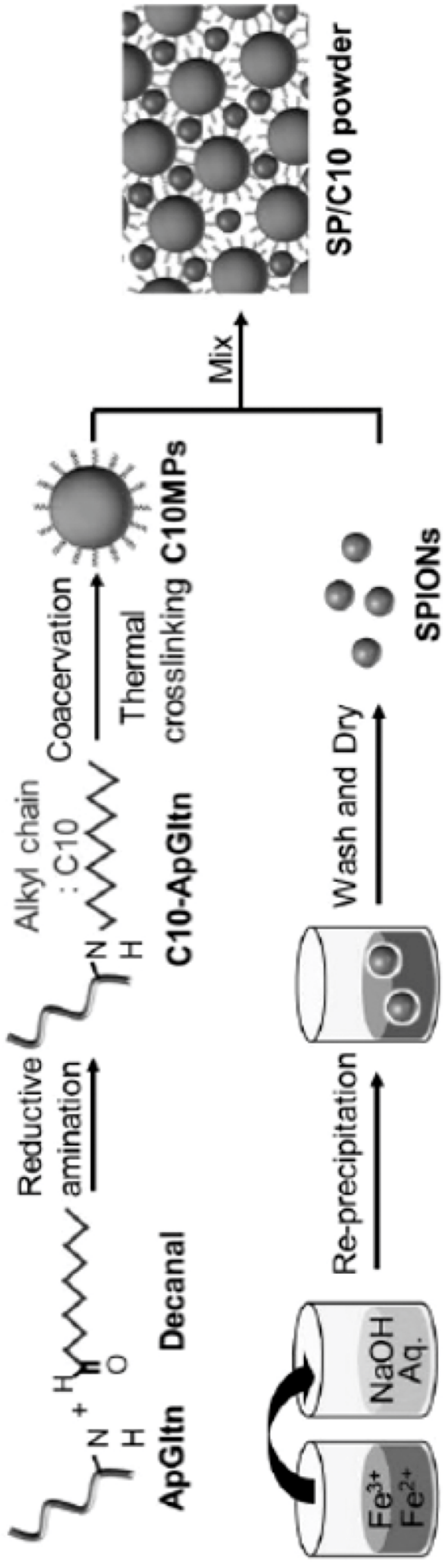
ApGltn
Decanal
Reductive
amination
Alkyl chain
: C10
C10-ApGltn
Coacervation
Thermal
crosslinking
C10MPs
NaOH
Aq.
Re-precipitation
Wash and Dry
SPIONs
Mix
SP/C10 powder

FIG. 5

FIG. 6

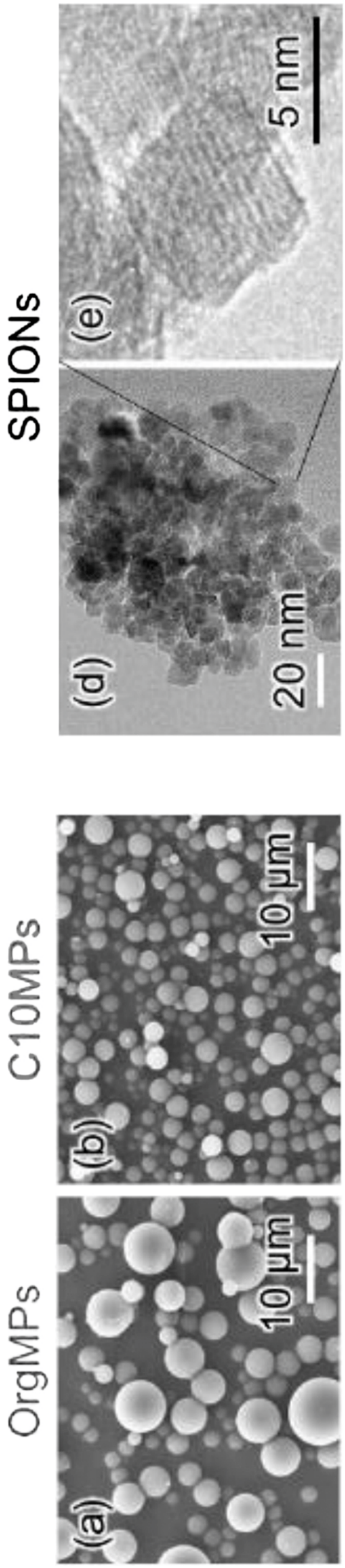

FIG. 7

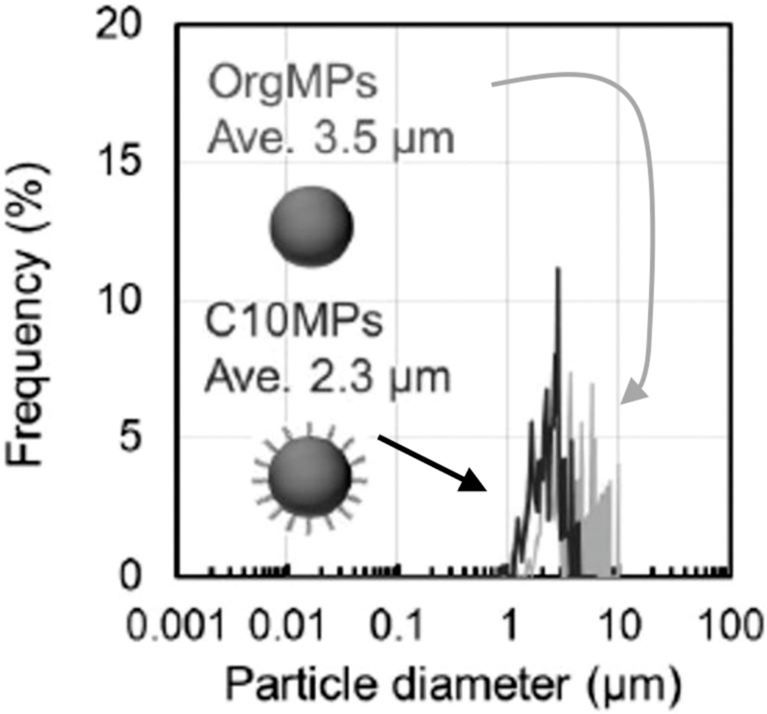
OrgMPs
Ave. 3.5 μm
C10MPs
Ave. 2.3 μm
Frequency (%)
Particle diameter (μm)
20
15
10
5
0
0.001
0.01
0.1
1
10
100

FIG. 8

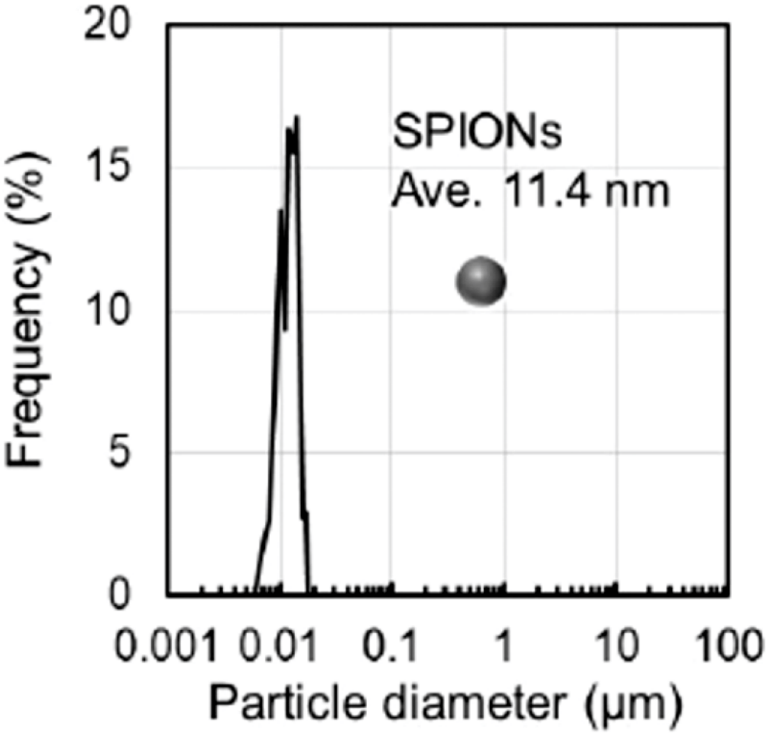
SPIONs
Ave. 11.4 nm
Frequency (%)
Particle diameter (μm)
20
15
10
5
0
0.001
0.01
0.1
1
10
100

FIG. 9

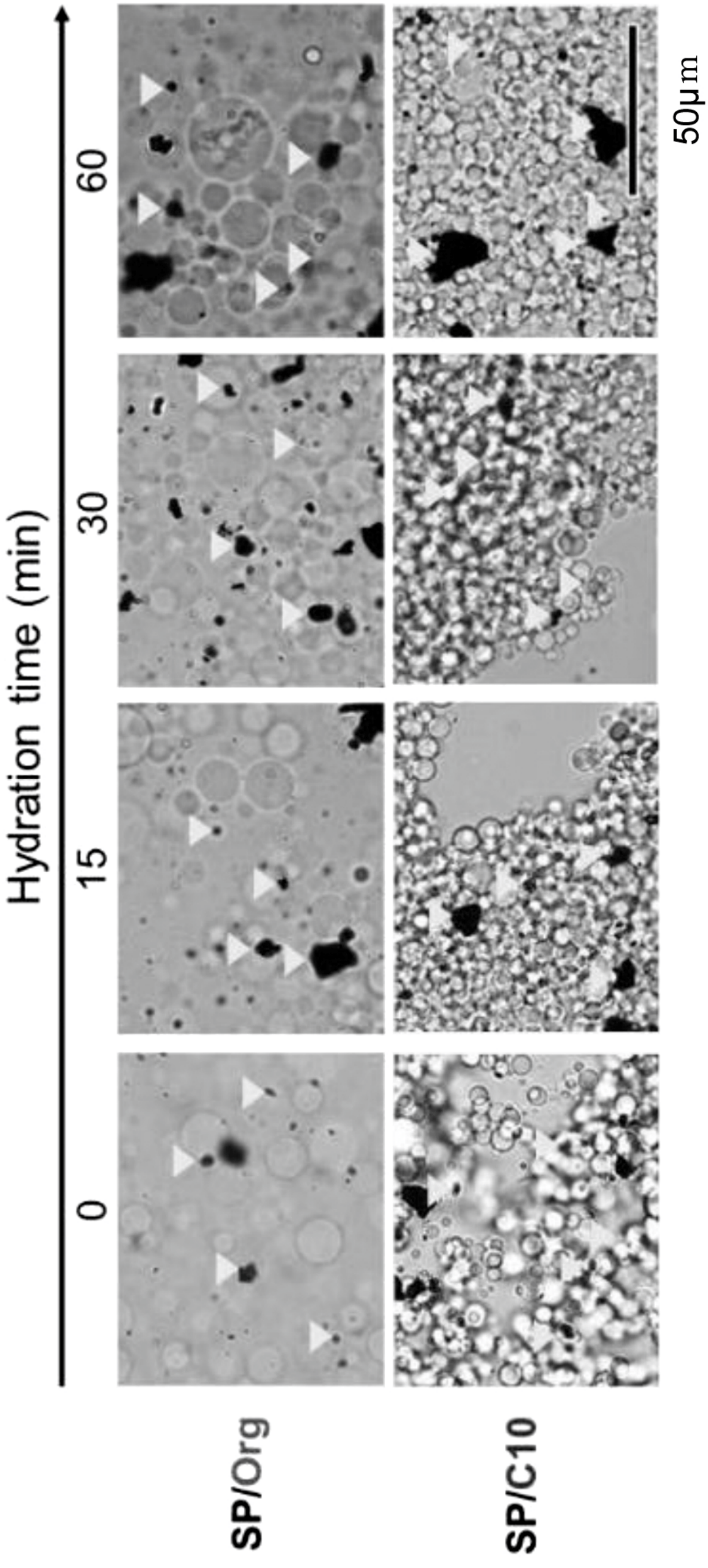

FIG. 10

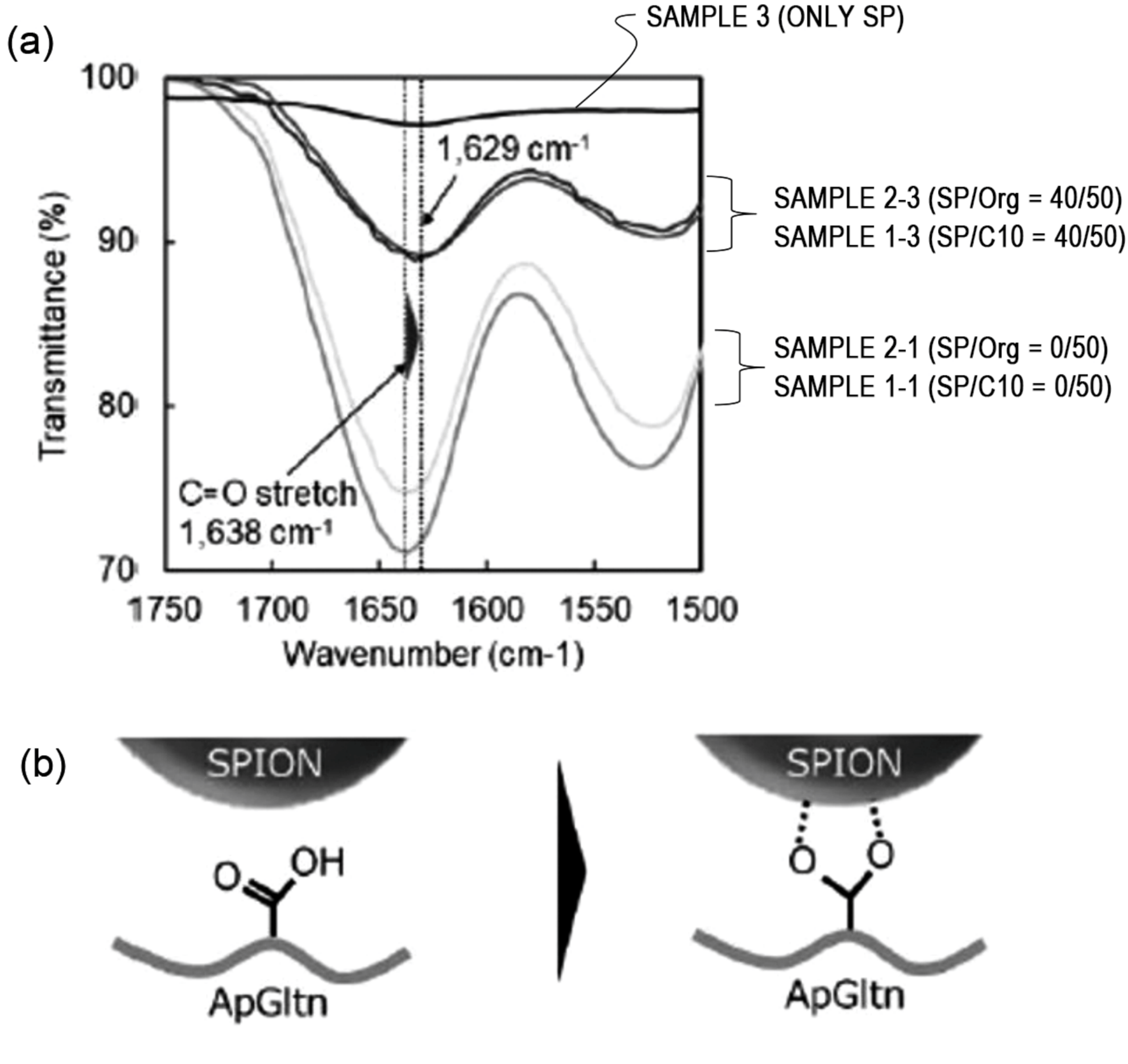
(a)
SAMPLE 3 (ONLY SP)
100
90
80
70
Transmittance (%)
1,629 cm-1
C=O stretch
1,638 cm-1
1750
1700
1650
1600
1550
1500
Wavenumber (cm-1)
SAMPLE 2-3 (SP/Org = 40/50)
SAMPLE 1-3 (SP/C10 = 40/50)
SAMPLE 2-1 (SP/Org = 0/50)
SAMPLE 1-1 (SP/C10 = 0/50)
(b)
SPION
OH
O
ApGltn
SPION
O
O
ApGltn

FIG. 11

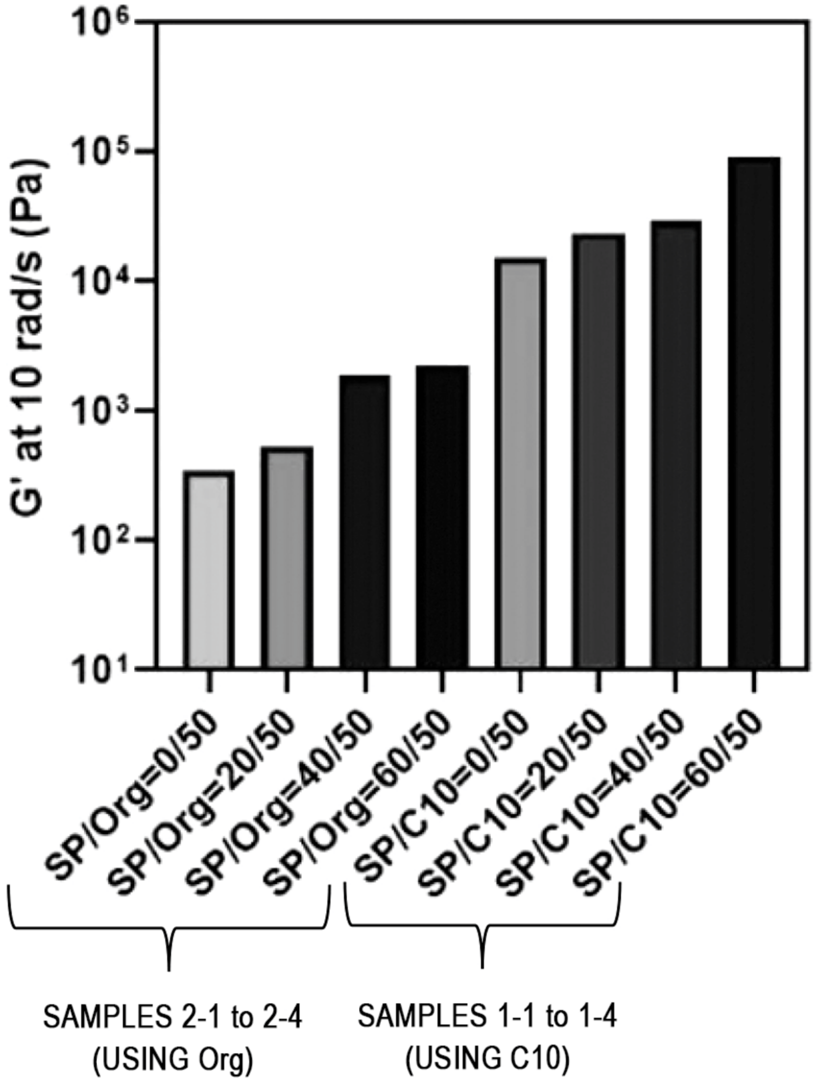

10⁶
10⁵
10⁴
10³
10²
10¹
G' at 10 rad/s (Pa)
SP/Org=0/50
SP/Org=20/50
SP/Org=40/50
SP/Org=60/50
SP/C10=0/50
SP/C10=20/50
SP/C10=40/50
SP/C10=60/50
SAMPLES 2-1 to 2-4
(USING Org)
SAMPLES 1-1 to 1-4
(USING C10)

FIG. 12

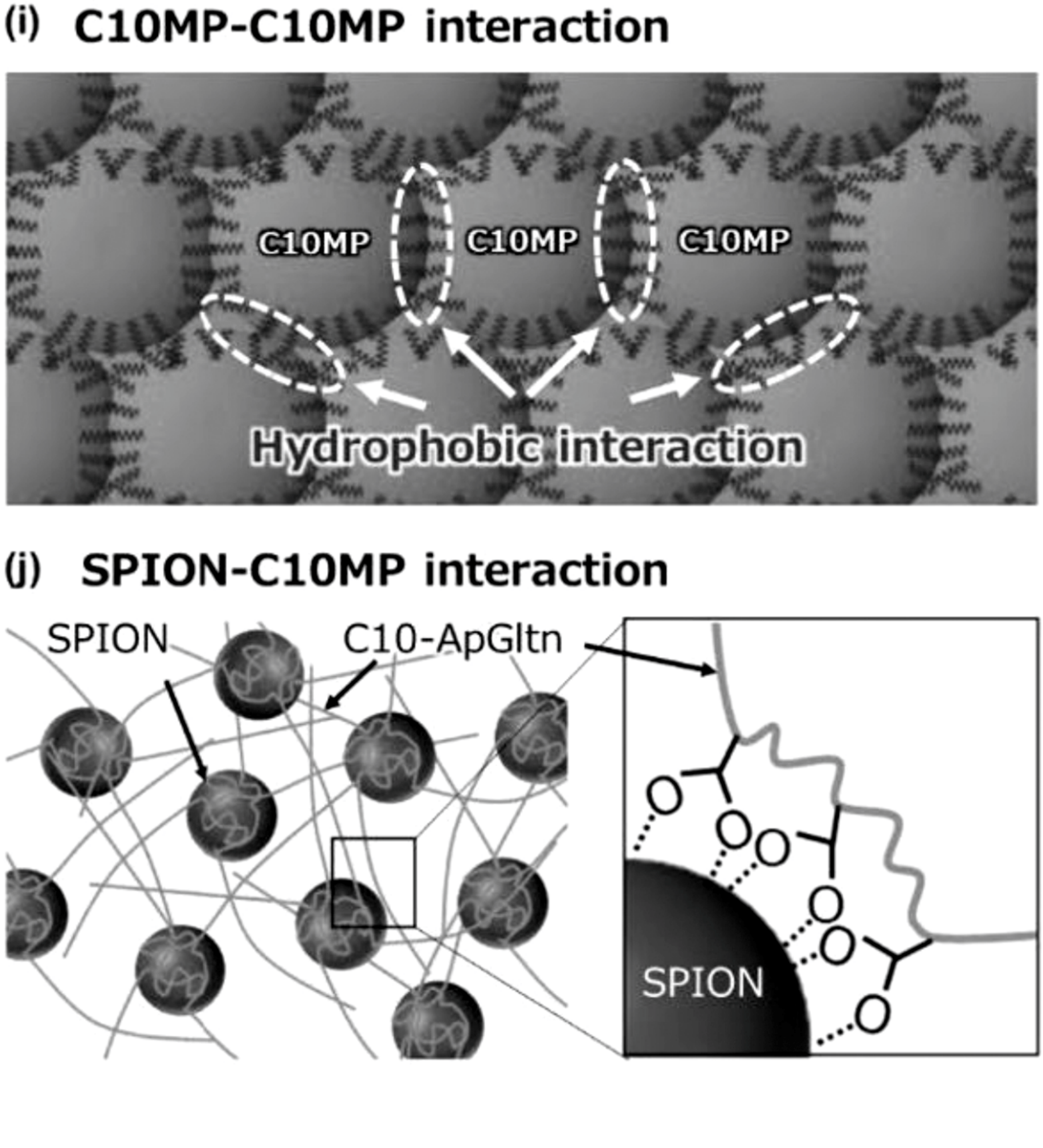
(i) C10MP-C10MP interaction
C10MP
C10MP
C10MP
Hydrophobic interaction
(j) SPION-C10MP interaction
SPION
C10-ApGltn
O
O
O
O
O
O
SPION

FIG. 13

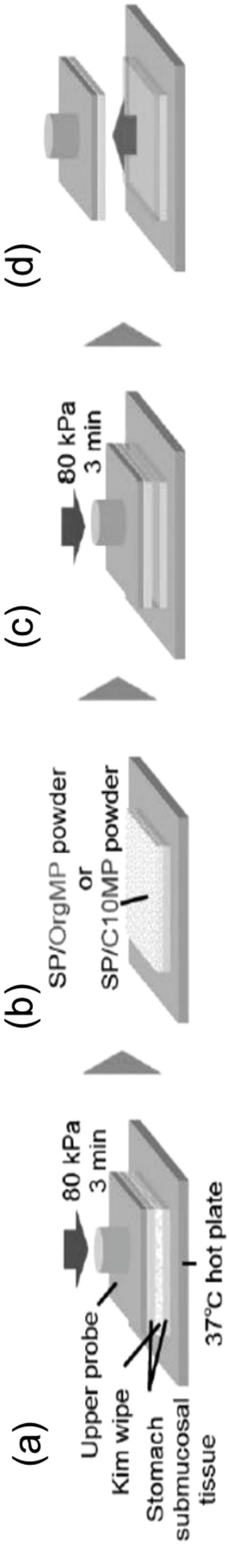
(a)
Upper probe
Kim wipe
Stomach submucosal tissue
80 kPa
3 min
37°C hot plate
(b)
SP/OrgMP powder
or
SP/C10MP powder
(c)
80 kPa
3 min
(d)

FIG. 14

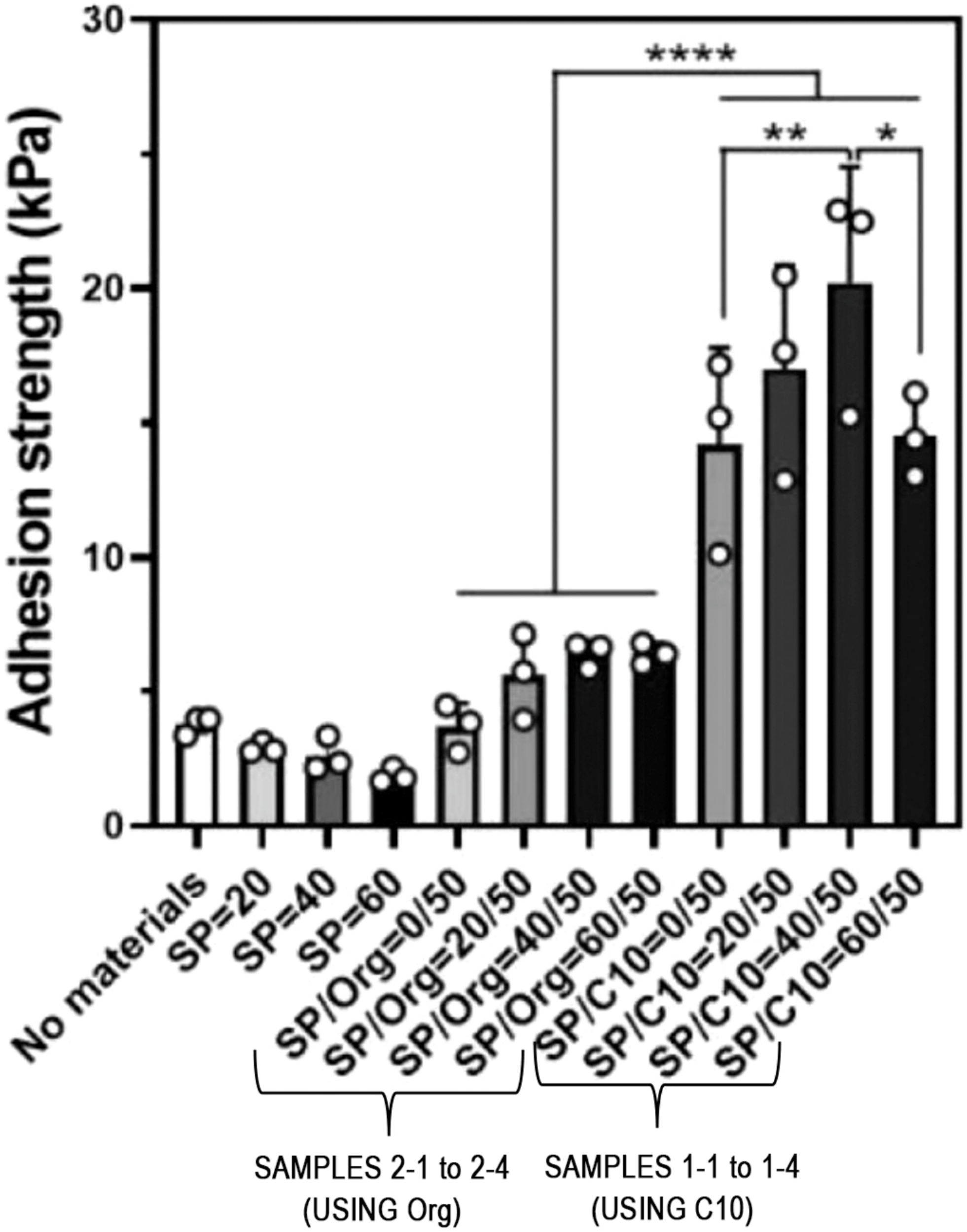
Adhesion strength (kPa)
0
10
20
30
****
**
*
No materials
SP=20
SP=40
SP=60
SP/Org=0/50
SP/Org=20/50
SP/Org=40/50
SP/Org=60/50
SP/C10=0/50
SP/C10=20/50
SP/C10=40/50
SP/C10=60/50
SAMPLES 2-1 to 2-4
(USING Org)
SAMPLES 1-1 to 1-4
(USING C10)

FIG. 15

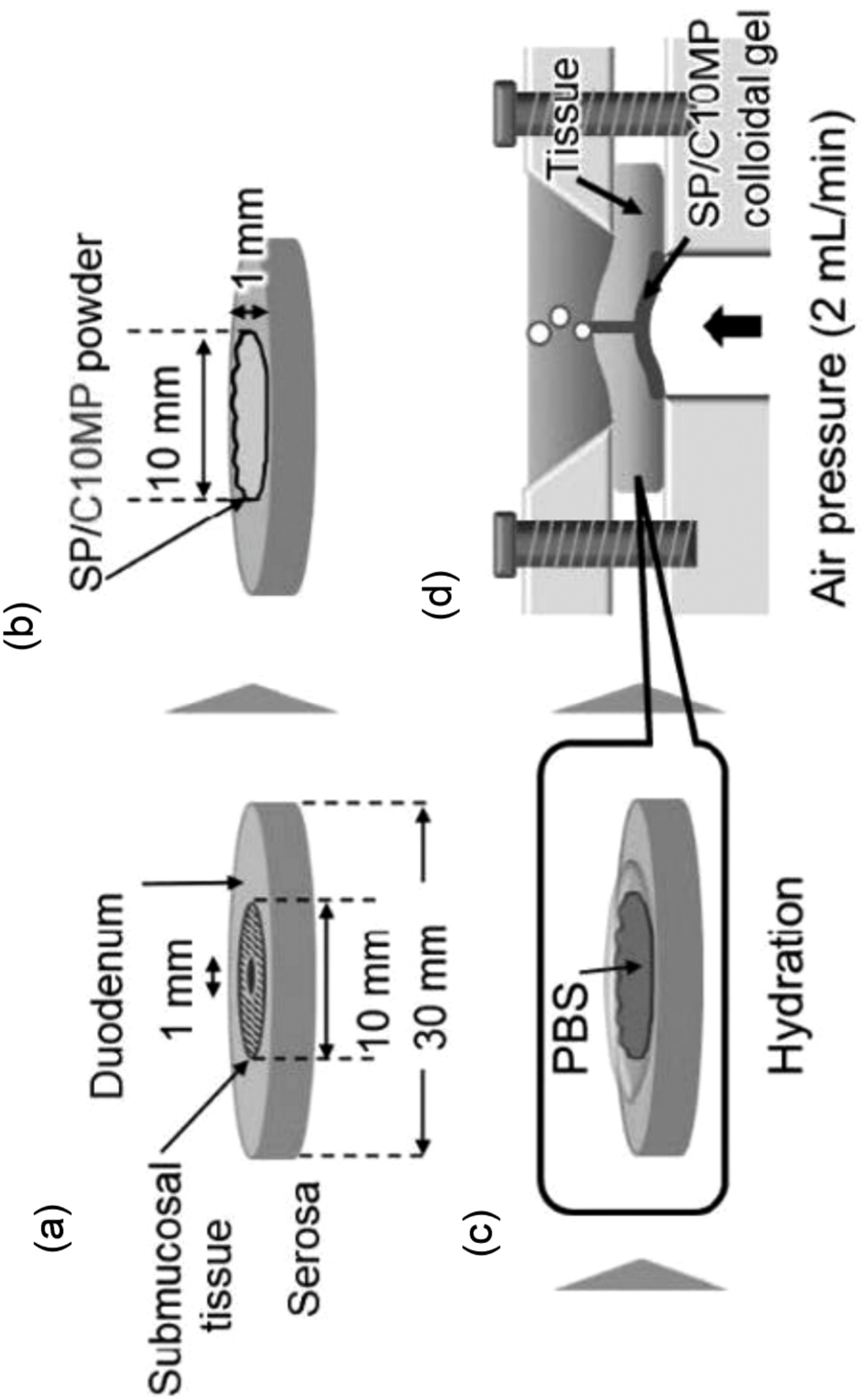
(a)
Submucosal tissue
Serosa
Duodenum
1 mm
10 mm
30 mm
(b)
SP/C10MP powder
10 mm
1 mm
(c)
PBS
Hydration
(d)
Tissue
SP/C10MP colloidal gel
Air pressure (2 mL/min)

FIG. 16

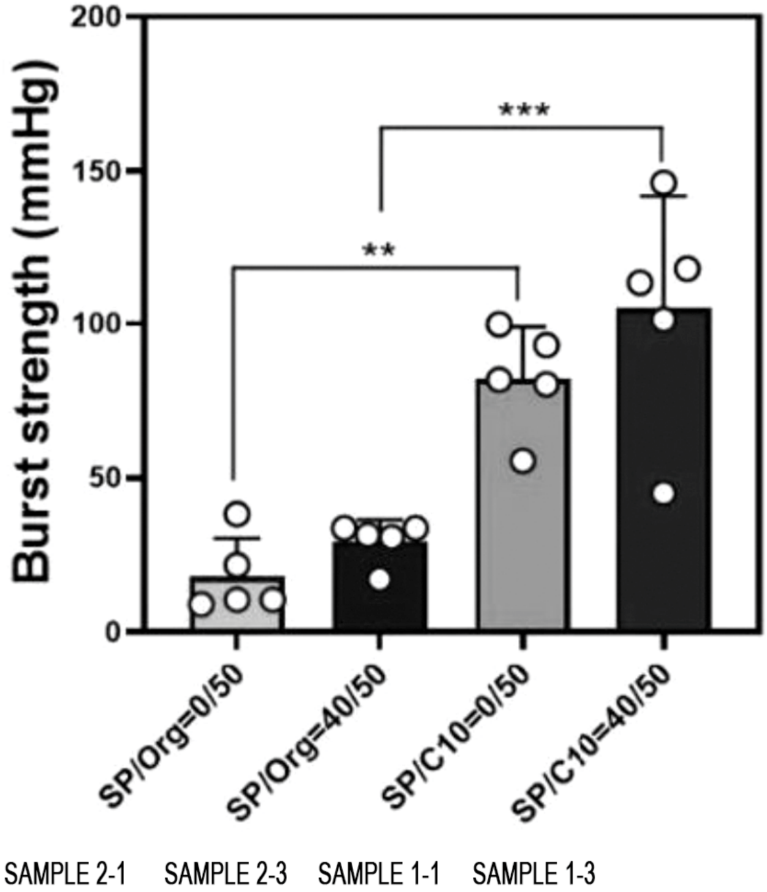
Burst strength (mmHg)
200
150
100
50
0
***
**
SP/Org=0/50
SP/Org=40/50
SP/C10=0/50
SP/C10=40/50
SAMPLE 2-1
SAMPLE 2-3
SAMPLE 1-1
SAMPLE 1-3

FIG. 17

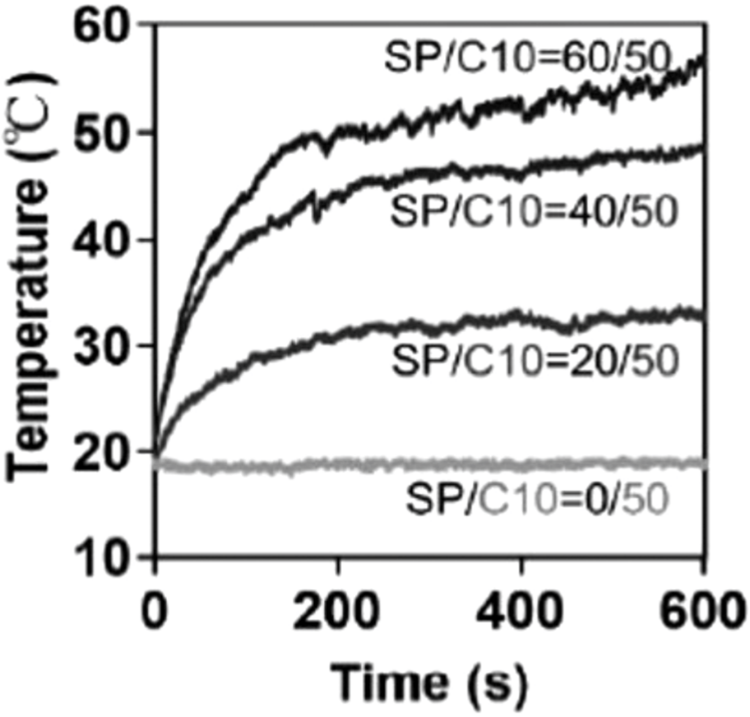
Temperature (℃)
60
50
40
30
20
10
0
200
400
600
Time (s)
SP/C10=60/50
SP/C10=40/50
SP/C10=20/50
SP/C10=0/50

FIG. 18

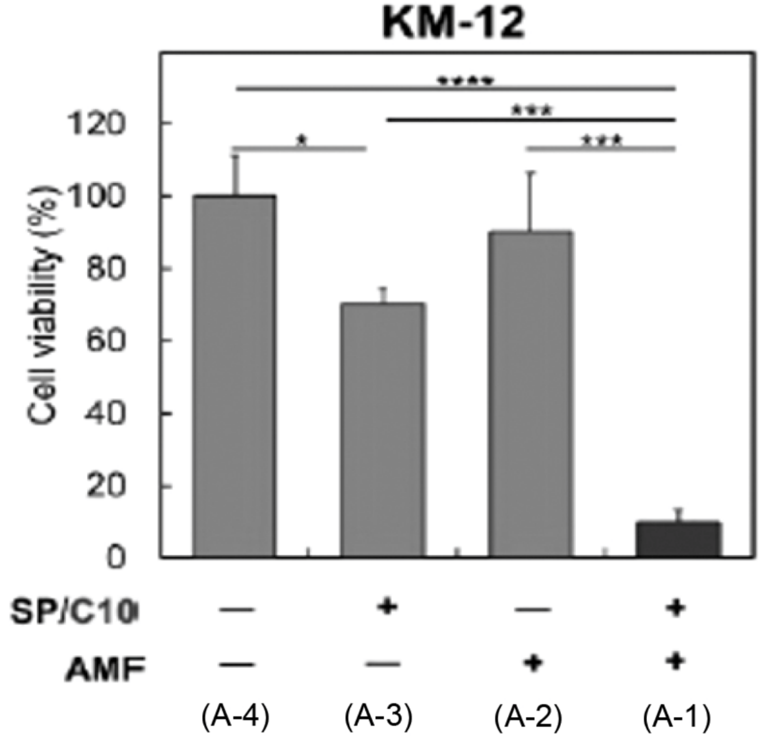
KM-12
Cell viability (%)
0
20
40
60
80
100
120
*
***
***
****
SP/C10 — + — +
AMF — — + +
(A-4) (A-3) (A-2) (A-1)

FIG. 19

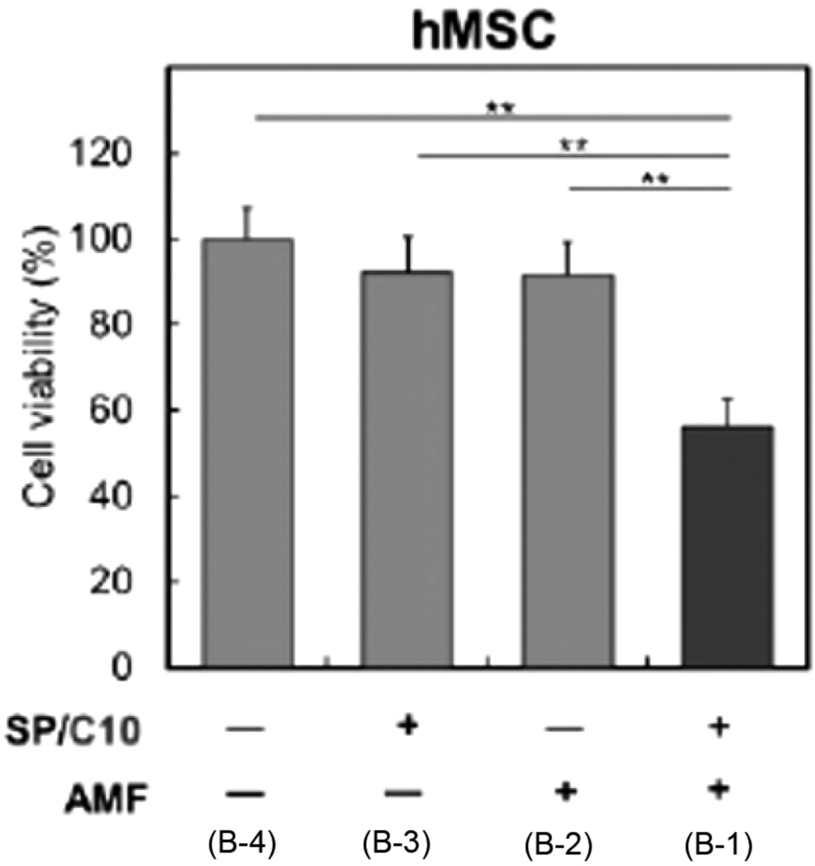
hMSC
Cell viability (%)
0
20
40
60
80
100
120
**
**
**
SP/C10 — + — +
AMF — — + +
(B-4) (B-3) (B-2) (B-1)

FIG. 20

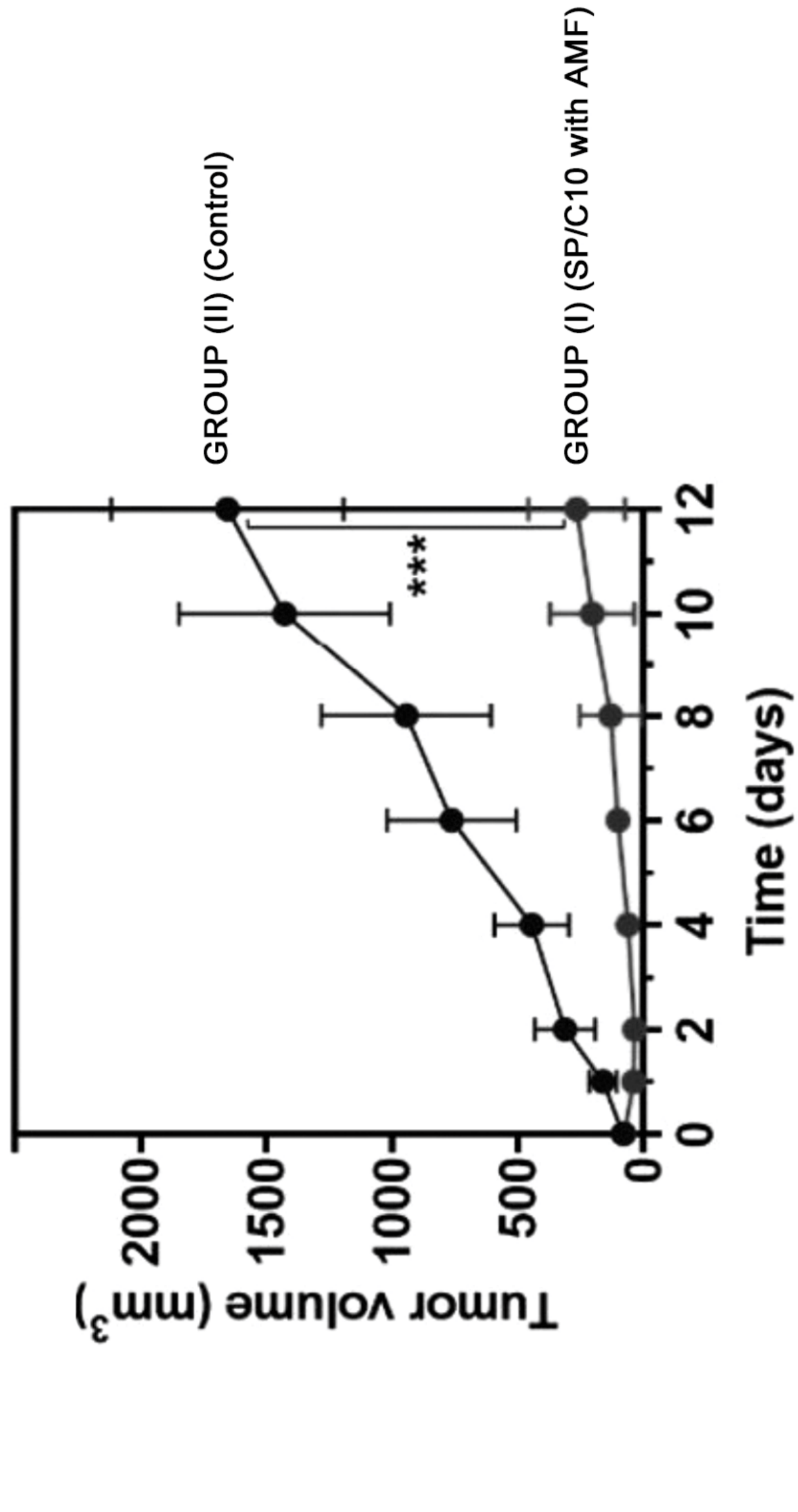
GROUP (II) (Control)
GROUP (I) (SP/C10 with AMF)
***
Tumor volume (mm$^3$)
0
500
1000
1500
2000
0
2
4
6
8
10
12
Time (days)

FIG. 21

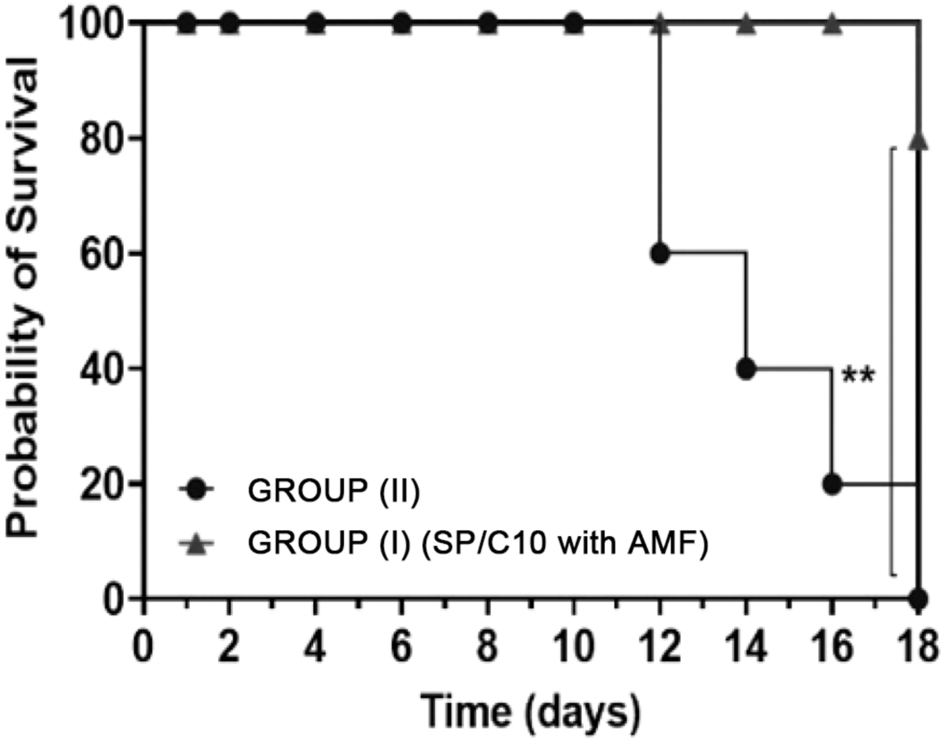
Probability of Survival
100
80
60
40
20
0
0 2 4 6 8 10 12 14 16 18
Time (days)
**
GROUP (II)
GROUP (I) (SP/C10 with AMF)

FIG. 22

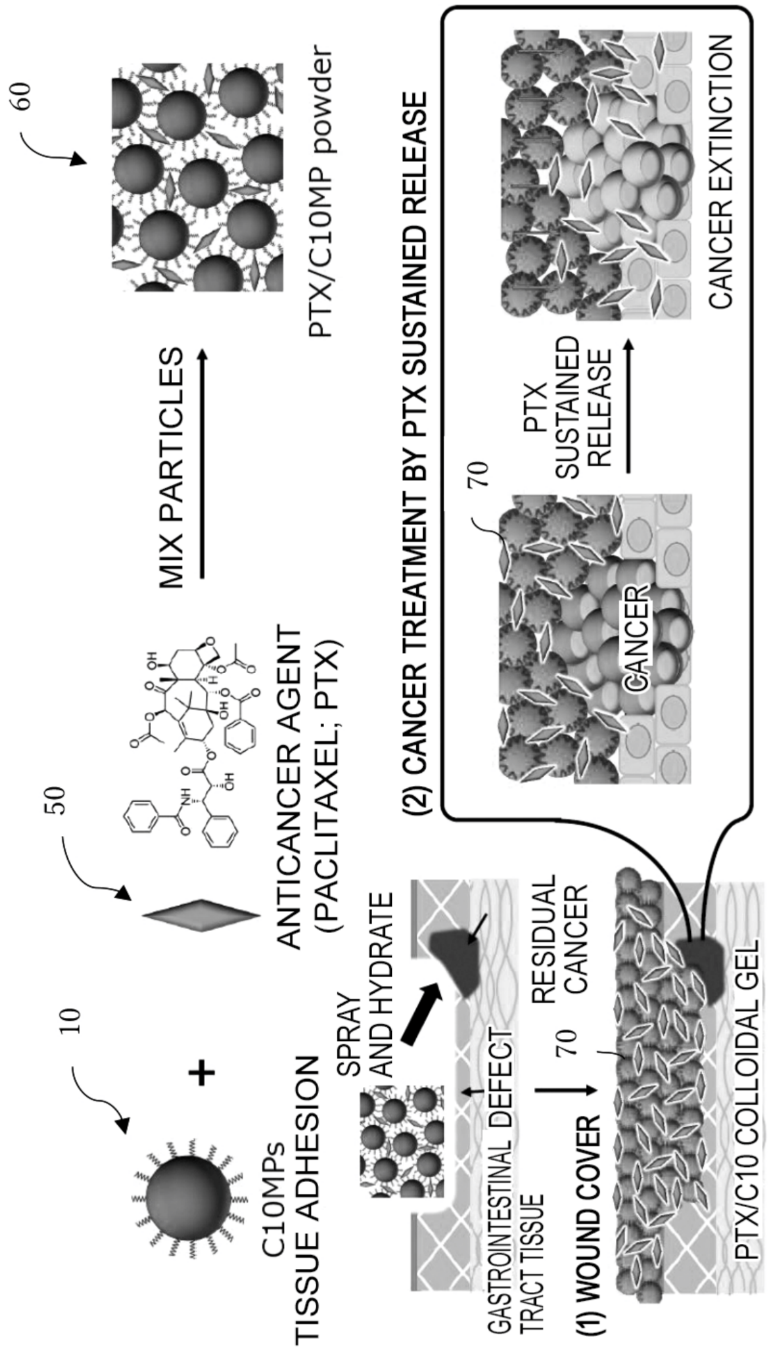
10
C10MPs
TISSUE ADHESION
+
50
ANTICANCER AGENT
(PACLITAXEL; PTX)
MIX PARTICLES
60
PTX/C10MP powder
SPRAY
AND HYDRATE
GASTROINTESTINAL DEFECT
TRACT TISSUE
RESIDUAL
CANCER
70
(1) WOUND COVER
PTX/C10 COLLOIDAL GEL
(2) CANCER TREATMENT BY PTX SUSTAINED RELEASE
70
CANCER
PTX
SUSTAINED
RELEASE
CANCER EXTINCTION

FIG. 23

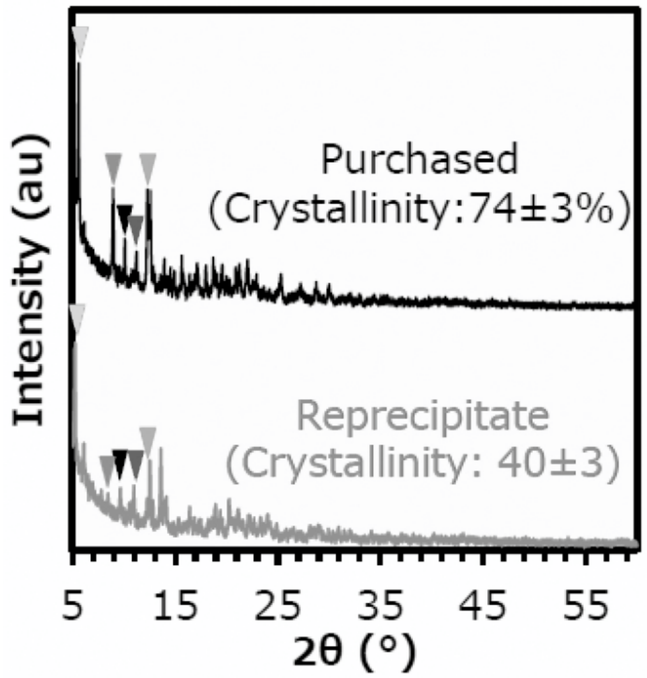
Intensity (au)
Purchased
(Crystallinity:74±3%)
Reprecipitate
(Crystallinity: 40±3)
5
15
25
35
45
55
2θ (°)

FIG. 24

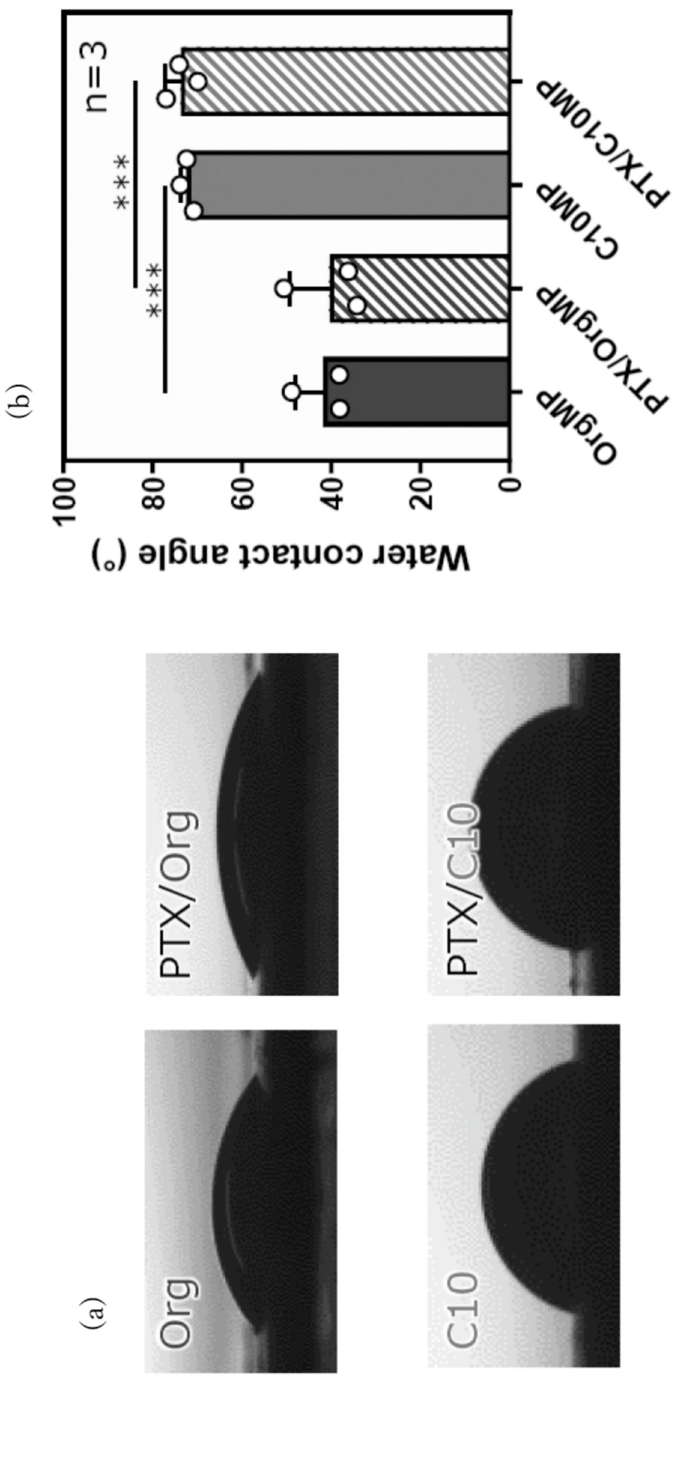
(a)
Org
PTX/Org
C10
PTX/C10
(b)
Water contact angle (°)
100
80
60
40
20
0
OrgMP
PTX/OrgMP
C10MP
PTX/C10MP
n=3
***
***

FIG. 25

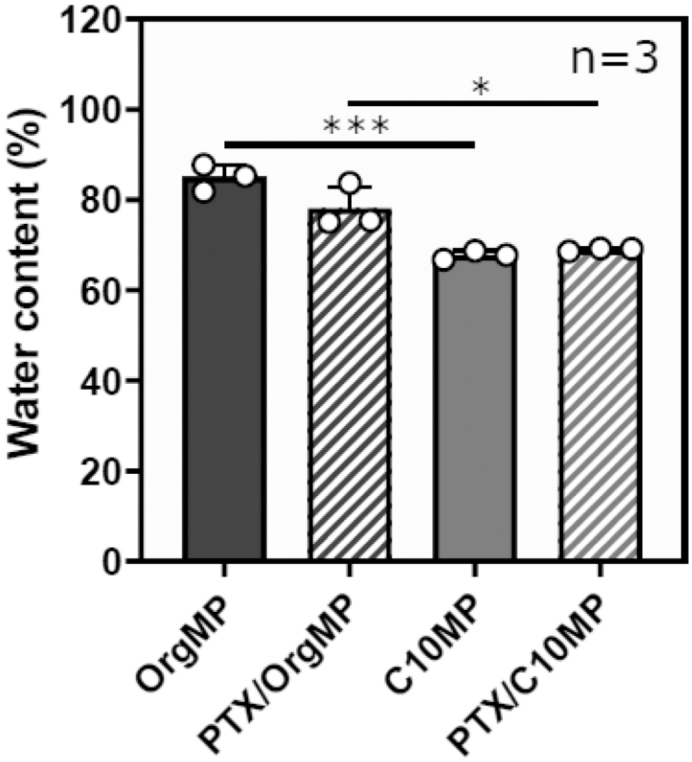
120
100
80
60
40
20
0
Water content (%)
n=3
*
***
OrgMP
PTX/OrgMP
C10MP
PTX/C10MP

FIG. 26

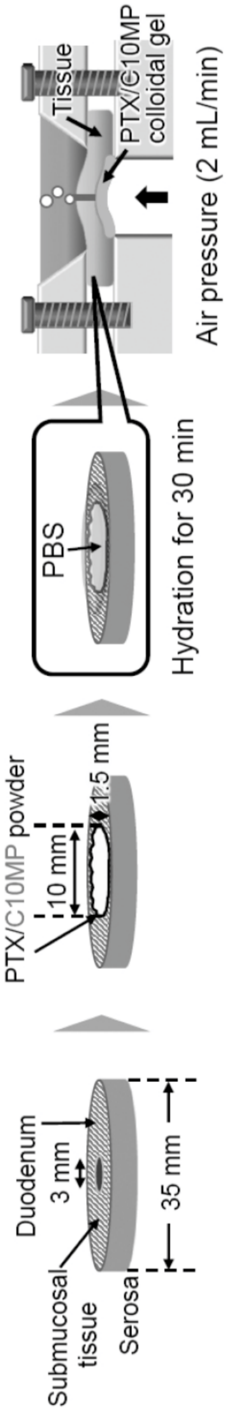
Submucosal tissue
Serosa
Duodenum
3 mm
35 mm
PTX/C10MP powder
10 mm
1.5 mm
PBS
Hydration for 30 min
Tissue
PTX/C10MP colloidal gel
Air pressure (2 mL/min)

FIG. 27

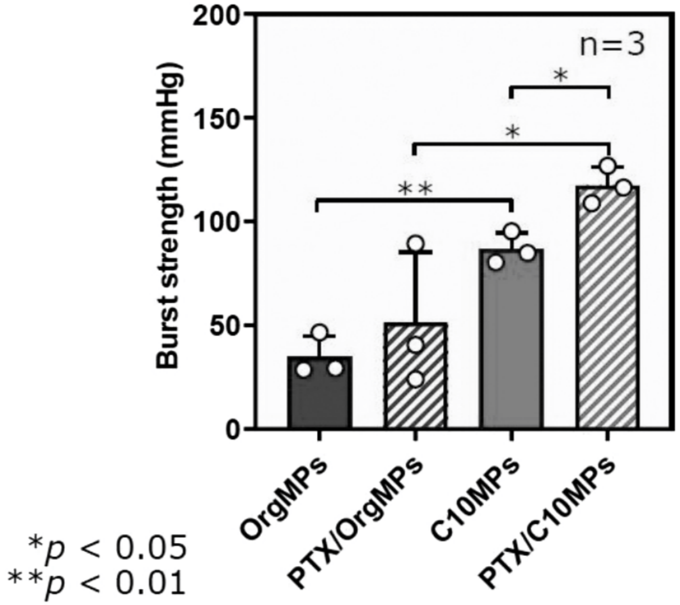
Burst strength (mmHg)
200
150
100
50
0
n=3
*
*
**
OrgMPs
PTX/OrgMPs
C10MPs
PTX/C10MPs
*p < 0.05
**p < 0.01

(a)

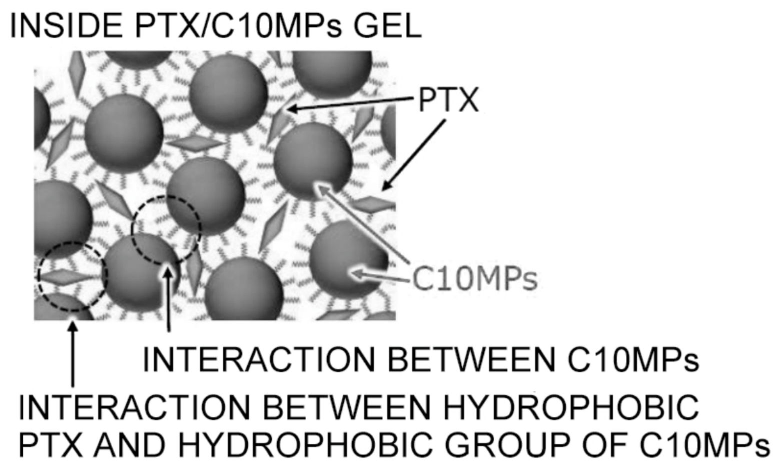
INSIDE PTX/C10MPs GEL
PTX
C10MPs
INTERACTION BETWEEN C10MPs
INTERACTION BETWEEN HYDROPHOBIC
PTX AND HYDROPHOBIC GROUP OF C10MPs

(b)

FIG. 28

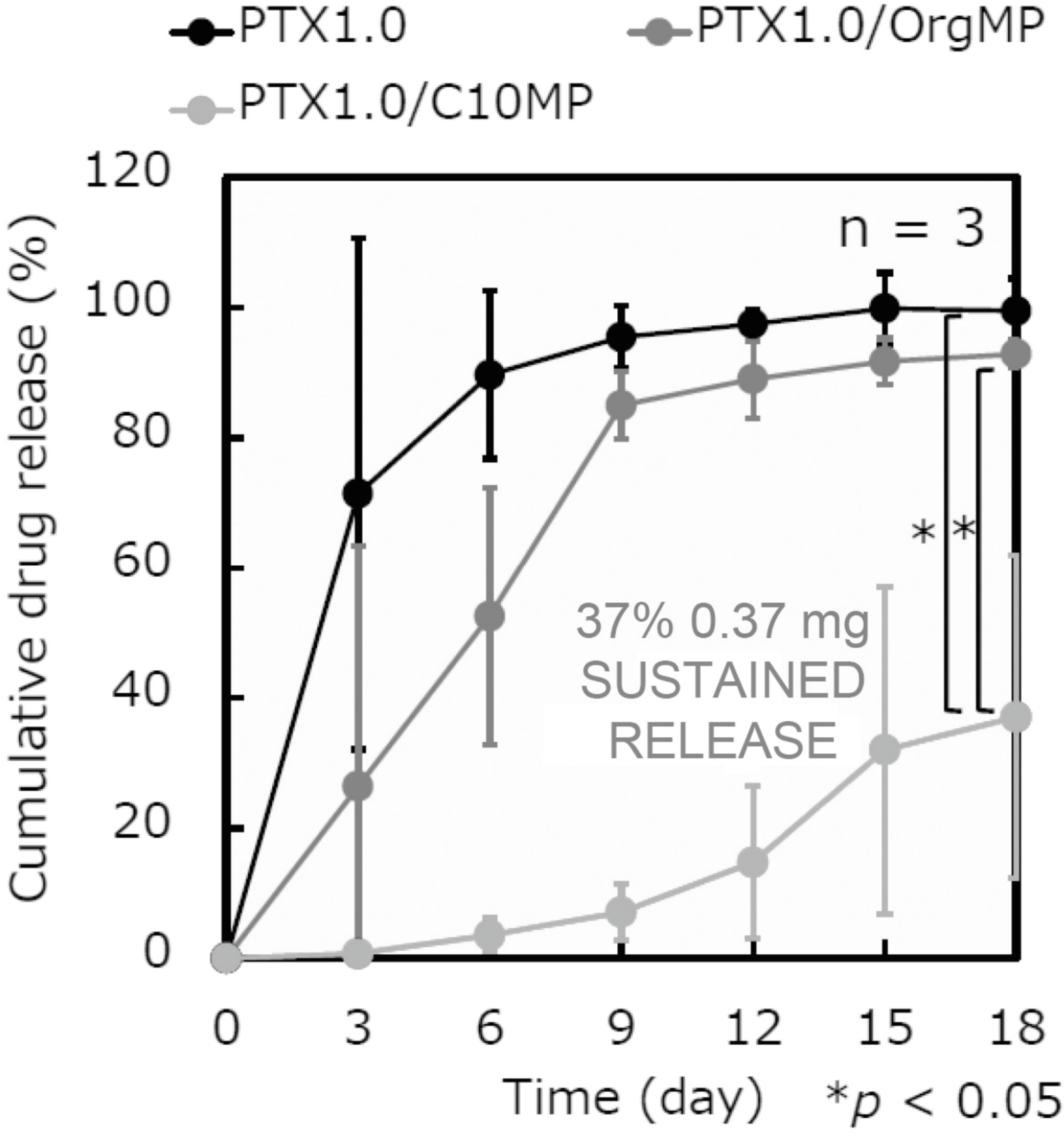
PTX1.0
PTX1.0/OrgMP
PTX1.0/C10MP
120
100
80
60
40
20
0
Cumulative drug release (%)
n = 3
37% 0.37 mg
SUSTAINED
RELEASE
* *
0
3
6
9
12
15
18
Time (day)
*p < 0.05

FIG. 29

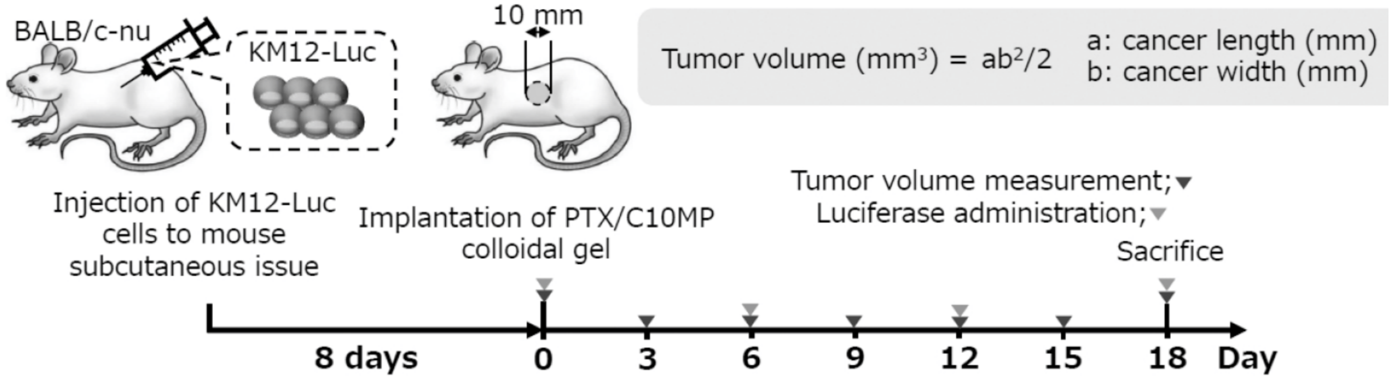
BALB/c-nu
KM12-Luc
10 mm
Tumor volume (mm³) = ab²/2
a: cancer length (mm)
b: cancer width (mm)
Injection of KM12-Luc cells to mouse subcutaneous issue
Implantation of PTX/C10MP colloidal gel
Tumor volume measurement;
Luciferase administration;
Sacrifice
8 days
0
3
6
9
12
15
18
Day

FIG. 30

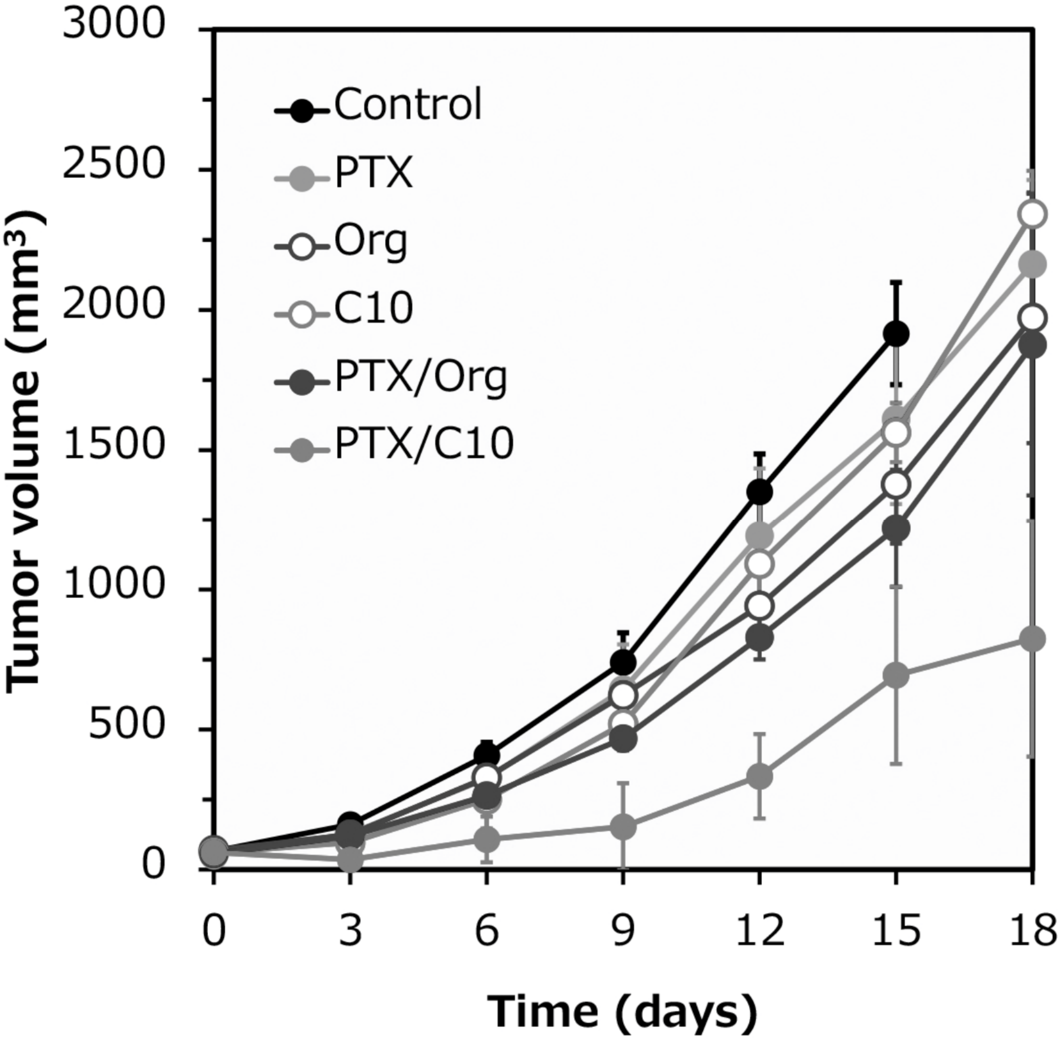

3000
2500
2000
1500
1000
500
0
Tumor volume (mm³)
Control
PTX
Org
C10
PTX/Org
PTX/C10
0
3
6
9
12
15
18
Time (days)

## INTERNATIONAL SEARCH REPORT

International application No.
**PCT/JP2024/024585**

**A. CLASSIFICATION OF SUBJECT MATTER**

***C07K 14/78***(2006.01)i; ***A61K 9/14***(2006.01)i; ***A61K 33/08***(2006.01)i; ***A61K 47/02***(2006.01)i; ***A61K 47/42***(2017.01)i; ***A61L 15/08***(2006.01)i; ***A61L 15/12***(2006.01)i; ***A61L 15/18***(2006.01)i; ***A61L 15/32***(2006.01)i; ***A61L 27/02***(2006.01)i; ***A61L 27/12***(2006.01)i; ***A61L 27/22***(2006.01)i; ***A61P 35/00***(2006.01)i

FI: C07K14/78; A61L15/32 100; A61L15/08; A61L15/12; A61L15/18; A61L27/02; A61L27/12; A61L27/22; A61K9/14; A61K47/42; A61K47/02; A61K33/08; A61P35/00

According to International Patent Classification (IPC) or to both national classification and IPC

**B. FIELDS SEARCHED**

Minimum documentation searched (classification system followed by classification symbols)

C07K14/78; A61K9/14; A61K33/08; A61K47/02; A61K47/42; A61L15/08; A61L15/12; A61L15/18; A61L15/32; A61L27/02; A61L27/12; A61L27/22; A61P35/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2022-126909 A (NATIONAL INSTITUTE FOR MATERIALS SCIENCE) 31 August 2022 (2022-08-31)<br>claims, paragraph [0041], examples | 1-54 |
| A | WO 2020/137903 A1 (NATIONAL INSTITUTE FOR MATERIALS SCIENCE) 02 July 2020 (2020-07-02)<br>claims, examples | 1-54 |
| A | WO 2019/045081 A1 (NATIONAL INSTITUTE FOR MATERIALS SCIENCE) 07 March 2019 (2019-03-07)<br>claims, examples | 1-54 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 August 2024** | **27 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

**INTERNATIONAL SEARCH REPORT**

International application No.
**PCT/JP2024/024585**

**C. DOCUMENTS CONSIDERED TO BE RELEVANT**

| Category* | Citation of document, with indication, where appropriate, of the relevant passages | Relevant to claim No. |
|---|---|---|
| A | 西口昭広、他, 組織接着性バイオポリマー微粒子の設計と医療応用, 色材協会誌, 2022, vol. 95, no. 11, pp. 341-345, (NISHIGUCHI, Akihiro et al. Design of a Biopolymer-Based Tissue Adhesive Particle for Biomedical Applications. Journal of the Japan Society of Colour Material.)<br>entire text | 1-54 |
| A | 田口哲志, 生体組織に接着する材料の設計と医学応用, 日本接着学会誌, 01 April 2023, vol. 59, no. 4, pp. 97-103, (TANIGUCHI, Tetsushi. Design of Tissue Adhesive Materials for Biomedical Applications. Journal of the Adhesion Society of Japan.)<br>summary, 5. Tissue-adhesive microparticles | 1-54 |
| A | 西口昭広、他, 消化器ガン治療を指向した多機能性創傷治癒粒子の創出, 第４０回日本バイオマテリアル学会大会予稿集, November 2018, 1B-06, (NISHIGUCHI, Akihiro et al.), non-official translation (Creation of multifunctional wound-healing particles aimed at gastrointestinal cancer treatment. Preprints the 40th Annual Meeting of the Japanese Society for Biomaterials.)<br>entire text | 1-54 |
| A | WO 2015/076252 A1 (NATIONAL INSTITUTE FOR MATERIALS SCIENCE) 28 May 2015 (2015-05-28)<br>claims, examples 1-17 | 1-54 |
| A | JP 2014-058465 A (NITTO DENKO CORPORATION) 03 April 2014 (2014-04-03)<br>claims, examples 1-9 | 1-54 |
| A | JP 2010-540513 A (BAR-ILAN UNIVERSITY) 24 December 2010 (2010-12-24)<br>claims, examples 1-8 | 1-54 |

Form PCT/ISA/210 (second sheet) (July 2022)

**INTERNATIONAL SEARCH REPORT**
**Information on patent family members**

International application No.
**PCT/JP2024/024585**

| Patent document cited in search report | Publication date (day/month/year) | Patent family member(s) | Publication date (day/month/year) |
|---|---|---|---|
| JP 2022-126909 A | 31 August 2022 | (Family: none) | |
| WO 2020/137903 A1 | 02 July 2020 | US 2022/0062179 A1<br>claims, examples<br>EP 3904377 A1<br>CN 113227129 A | |
| WO 2019/045081 A1 | 07 March 2019 | US 2020/0206382 A1<br>claims, examples<br>EP 3679954 A1<br>CN 111065421 A | |
| WO 2015/076252 A1 | 28 May 2015 | (Family: none) | |
| JP 2014-058465 A | 03 April 2014 | US 2014/0079794 A1<br>claims, examples 1-9<br>EP 2708570 A1<br>CN 103656625 A | |
| JP 2010-540513 A | 24 December 2010 | US 2009/0110644 A1<br>claims, examples 1-8<br>WO 2009/040811 A2<br>EP 2205282 A2 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020137903 A
- JP 7132465 B
- JP 2007231225 A